# EUROPEAN PATENT APPLICATION

(11) **EP 2 649 878 A1**
(43) Date of publication of application: **16.10.2013**
(21) Application number: 13003432.5
(22) Date of filing: 25.03.2011
(51) Int. Cl.: A01N 25/10, A01N 59/00, A01N 59/16, A01N 31/16, A01N 33/12, A01N 37/36, A61L 15/46, A61L 27/34, A61K 9/70, B32B 27/30, B32B 27/32, C09D 131/04, C09D 5/14, C08J 9/04, A01N 25/34, C09D 5/16, B32B 27/08, B32B 27/18, B32B 3/30

(54) **Methods for increasing effectiveness of antimicrobial agents in polymeric films**

(30) Priority: 14.04.2010 US 323890 P; 23.12.2010 US 201061426914 P
(62) Divisional of application: 11715108.4
(71) Applicant: AVERY DENNISON CORPORATION, Pasadena, CA 91103 (US)
(72) Inventor: Henderson, Kevin O., Willoughby Hills, Ohio 44094 (US)
(74) Representative: Müller-Boré & Partner Patentanwälte

(57) **Abstract**

The application relates: to multilayered polymeric films comprising a first skin layer comprising at least one gas and vapor barrier organic polymer, a core layer that comprises at least one elastomeric organic polymer, and a second skin layer that comprises at least one sealable organic polymer and at least one antimicrobial agent; to articles comprising the film useful in various applications such as medical and health care applications and to a method of providing a gas and vapor barrier with antimicrobial activity to an article comprising manufacturing an article comprising said film.

## Description

### Cross-Reference to Related Applications

The present application claims priority to U.S. Provisional Patent Application Nos. 61/323,890 filed April 14, 2010, and 61/426,914 filed December 23, 2010, both of which are incorporated herein by reference in their entireties.

### Field of the Invention

The present invention relates to polymeric films containing an antimicrobial agent, and articles and methods comprising the films. The invention also relates to methods for increasing the effectiveness of such agents incorporated into polymeric films. The films, articles, and methods thereof are useful in various applications such as medical and health care applications.

### Background of the Invention

Numerous applications are known in which a gas and vapor barrier film is incorporated in articles to prevent the transmission of gases and vapors to thereby control or prevent the loss of freshness and flavor and/or the escape of aroma or odor. In some applications, including medical and health care applications, microbial activity with concurrent microbial growth can lead to malodorous gases and vapors and possible infection from the related articles. Articles that involve microbial activity usually employ a passive gas and vapor barrier film to contain malodorous gases and vapors. Venting of built up gases and vapors is required in some of these articles, such as for example ostomy pouches.

Silver ions are highly effective as an antimicrobial agent. However, compounds containing such ions are also relatively expensive. Most antimicrobial technologies using silver ions are limited by the amount of silver ion that can reach the surface of a polymeric film for exposure to microbes. Higher concentrations of silver ions in a polymeric film have limited effects, as the ions tend to block each other and prevent exposure of additional ions below the surface. Accordingly, a need exists for a strategy by which the antimicrobial efficacy of silver ion along a face of a film can be increased.

### Summary of the Invention

The difficulties and drawbacks associated with previously known films and practices associated with antimicrobial agents are addressed in the present polymeric films, articles and methods comprising the films.

In one aspect, the present invention provides a polymeric composition adapted for forming a film. The composition comprises at least one thermoplastic polymer, an antimicrobial agent, and an antimicrobial agent promoter selected from the group consisting of (i) at least one blowing agent, (ii) at least one hydrophilic wetting agent, and (iii) combinations of (i) and (ii).

In another aspect, the present invention provides a polymeric film comprising at least one thermoplastic polymer and an antimicrobial agent. The film satisfies at least one of conditions (a) and (b) in which condition (a) is that the film also comprises an antimicrobial agent promoter which includes at least one hydrophilic wetting agent, and condition (b) is that the film initially comprised an antimicrobial agent promoter that included at least one blowing agent which subsequently formed bubbles within the film and ruptured along a face of the film, to thereby form a cratered surface on the film.

In still another aspect, the present invention provides a method for increasing the effectiveness of an antimicrobial agent incorporated in a polymeric composition. The method comprises providing a polymeric composition including at least one thermoplastic polymer, and an antimicrobial agent. The method also comprises adding to the composition an antimicrobial agent promoter selected from the group consisting of (i) at least one blowing agent, (ii) at least one hydrophilic wetting agent, and (iii) combinations of (i) and (ii) to thereby form an improved polymeric composition.

In still another aspect, the present invention provides a multilayer barrier film comprising an odor barrier composite including layers of a norbornene-based material and/or ethylene vinyl alcohol. The multilayer barrier film also comprises a polymeric film including at least one thermoplastic polymer, and an antimicrobial agent. The film satisfies at least one of conditions (a) and (b) in which condition (a) is that the film also comprises an antimicrobial agent promoter which includes at least one hydrophilic wetting agent, and condition (b) is that the film initially comprised an antimicrobial agent promoter that included at least one blowing agent which subsequently formed bubbles within the film and ruptured along a face of the film, to thereby form a cratered surface on the film.

In another aspect, the present invention provides a multilayered polymeric film which comprises a first skin layer comprising at least one gas and vapor barrier organic polymer and having an upper surface and a lower surface. The multilayered film also comprises a core layer including at least one elastomeric organic polymer and having an upper surface and a lower surface where the upper surface of the core layer underlies the lower surface of the first skin layer. The multilayer film also comprises a second skin layer including at least one sealable organic polymer and at least one antimicrobial agent and having an upper surface and a lower surface where the upper surface of the second skin layer underlies the lower surface of the core layer.

In yet another aspect the invention provides the film, as described herein which, further comprises at least one tie layer comprising at least one adhesive organic polymer and having an upper surface and a lower surface where the upper surface of the tie layer underlies the lower surface of the first skin layer and the lower surface of the tie layer overlies the upper surface of the core layer or the upper surface of the tie layer underlies the lower surface of the core layer and the lower surface of the tie layer overlies the upper surface of the second skin layer.

An additional aspect of the invention is an article in which the article comprises the film as described herein.

In still another aspect, the present invention provides a method to provide a gas and vapor barrier and antimicrobial activity in an article manufactured from a film. The method comprises providing a film in which the film includes a first skin layer comprising at least one gas and a vapor barrier organic polymer and having an upper surface and a lower surface, a core layer comprising at least one elastomeric organic polymer and having an upper surface and a lower surface where the upper surface of the core layer underlies the lower surface of the first skin layer, and a second skin layer comprising at least one sealable polymer and at least one antimicrobial agent and having an upper surface and a lower surface where the upper surface of the second skin layer underlies the lower surface of the core layer. The method also comprises manufacturing an article where the article comprises the film.

As will be realized, the invention is capable of other and different embodiments and its several details are capable of modifications in various respects, all without departing from the invention. Accordingly, the drawings and description are to be regarded as illustrative and not restrictive.

### Brief Description of the Drawings

The appended drawings are provided to illustrate and not limit the present invention. The components in the drawings are not to scale with emphasis instead to illustrating the principles of the invention. In the drawings the components of the film of the invention may have the same reference number throughout the drawings.

Figure 1 is a schematic perspective view of a preferred embodiment polymeric film according to the present invention.

Figure 2 is a schematic detailed cross sectional view of the preferred film illustrated in Figure 1.

Figure 3 is a schematic cross sectional view of a preferred embodiment multilayer assembly including the preferred film depicted in Figures 1 and 2.

Figure 4 is a graph of bacterial counts of various samples at different incubation times in Example 1.

Figure 5 is a cross sectional view of a film having three layers according to an embodiment of the invention.

Figure 6 is a cross sectional view of a film having four layers according to an embodiment of the invention.

Figure 7 is a cross sectional view of a film having four layers according to an embodiment of the invention.

Figure 8 is a cross sectional view of a film having five layers according to an embodiment of the invention.

### Detailed Description of the Embodiments

Generally, in accordance with the present invention, one or more antimicrobial promoters are utilized which when incorporated into a polymeric composition and a film is formed therefrom, significantly improve the antimicrobial activity of agent(s) in the compositions. The antimicrobial promoters preferably include blowing agents and/or hydrophilic wetting agents which are added to a polymeric composition containing an antimicrobial agent. The polymeric composition can be coextruded to form a polymeric film. The film or layer can be incorporated into a wide range of articles and preferably used in a multilayer barrier assembly. The polymeric layer to which the antimicrobial promoter is added, includes at least one antimicrobial agent such as but not limited to silver ion, zinc oxide, triclosan, and/or other antimicrobial agents. For embodiments in which the promoter includes blowing agent(s), the polymeric layer has a particular thickness that is preferably too thin to form foam, so the emerging bubbles rupture, thereby creating a much higher surface area along the face of the film. The increased surface area provides more regions for exposure of the antimicrobial agent and resulting contact with microbes. The higher surface area significantly increases the effectiveness of the antimicrobial agent at nearly any given concentration. For embodiments in which the antimicrobial promoter includes one or more hydrophilic wetting agents, the incorporation of a wetting agent increases contact between a microbe-containing aqueous medium and a face of the polymeric layer containing the antimicrobial agent(s). The present invention includes polymeric layers having antimicrobial agents in conjunction with (i) one or more blowing agents, and/or (ii) one or more hydrophilic wetting agents. That is, the invention includes the use and incorporation of each of (i) and (ii) separately and in combination with one another. These and other aspects are described in greater detail herein.

### Polymeric Film

In a preferred embodiment of the present invention the polymeric layer comprises at least one sealable organic polymer, at least one antimicrobial agent, and at least one antimicrobial agent promoter. The sealable organic polymer comprises any thermoplastic organic polymer that allows sealing of the polymeric layer to itself or another layer, to a layer of a different film, or to an article. In various embodiments of the invention the sealing method includes sealing with an adhesive such as for example a pressure sensitive adhesive or solvent adhesive, sealing via a lamination using a laminate adhesive and/or pressure and/or heat, sealing with heat, or any combination of the foregoing sealing methods. In one preferred embodiment, the sealing method is a heat sealing method. In certain embodiments the heat sealing method comprises sealing with a heated bar, sealing with hot air, sealing with an electromagnetic radiation source such as for example infrared radiation or radio frequency radiation, or a combination of any of the foregoing heat sealing methods.

The sealable organic polymer comprises a polyolefin, a polyester, a polyamide, a styrene-based homopolymer or copolymer, a polycarbonate, a polyurethane, a halogen-containing polymer, an acrylate-based or methacrylate-based homopolymer or copolymer, an alkene-unsaturated carboxylic acid or unsaturated carboxylic acid derivative copolymer, a vinyl alcohol-containing homopolymer or copolymer, or a mixture of two or more of any of the foregoing polymers. In certain embodiments of the invention, the sealable organic polymer is heat sealable and comprises a polyethylene, an alkene-unsaturated carboxylic acid or unsaturated carboxylic acid derivative copolymer, or a mixture of two or more of any of the foregoing polymers. Preferably, the polyethylene is heat sealable and comprises a high density polyethylene, a medium density polyethylene, a low density polyethylene, a linear low density polyethylene, a very low density polyethylene, a polyethylene plastomer, or a mixture of two or more of any of the foregoing polyethylenes. The alkene-unsaturated carboxylic acid or unsaturated carboxylic acid derivative copolymer is also preferably heat sealable and comprises an ethylene vinyl acetate copolymer, an ethylene-methacrylic acid copolymer, an ethylene-acrylic acid copolymer, an ethylene-alkyl methacrylate copolymer, an ethylene-alkyl acrylate copolymer, an ionomer to include metal salts of ethylene-methacrylic acid copolymers, or a mixture of two or more of any of the foregoing copolymers. In certain embodiments, the ethylene vinyl acetate copolymer is heat sealable and on a weight basis has a vinyl acetate content of 10% to 40%, 16% to 36%, or 22% to 32%. Generally, the sealable organic polymer is present on a weight basis in the polymeric layer at 20% to 70%, 30% to 60%, or 40% to 50%. Useful sealable organic polymers are commercially available and include ethylene vinyl acetate (EVA) copolymers ATEVA^{®} 2810A from Celanese which have an ASTM D 792 density of 0.949 g/cm³ and an ASTM D 1238 melt index at 190°C/2.16 kg of 6 dg/minute and a vinyl acetate content of 28% by weight, and ELVAX^{®} 3182 from DuPont^{™} which has an ASTM D 792 density of 0.95 g/cm³ and an ASTM D 1238 melt index at 190°C/2.16 kg of 3 dg/minute and a vinyl acetate content of 28% by weight.

In certain embodiments, it is preferred to include ethylene vinyl acetate in combination with a linear low density polyethylene, and most preferably with a metallocene-catalyzed linear low density polyethylene (LLDPE). Preferred proportions of these components are as follows. The ethylene vinyl acetate is preferably used in an amount of from about 40% to about 50%, and most preferably about 45%. And, the metallocene-catalyzed LLDPE is used in an amount of from about 6% to about 16%, and most preferably about 11%.

In addition to the blowing agent and/or the hydrophilic wetting agent, and the antimicrobial agent, the polymeric film may also comprise further additives, such as slip agents, blocking agents, nucleating agents, extrusion aids, antioxidants, flame retardants, colorants, or pigments. Examples of pigments are carbon black or titanium dioxide or graphite and other compounds known in the art.

Preferably, in certain embodiments for the polymeric layer, when utilizing one or more blowing agents for the antimicrobial promoter, it is preferred that the polymeric layer have a certain thickness to promote surface characteristics and maintain sealing. When the polymeric film is incorporated and used in a multilayer assembly such as in a multilayer barrier for medical applications, a preferred thickness is targeted between 5% to 15% of the overall film construction. Generally, the thickness of the polymeric layer is sufficiently thin such that the composition does not readily form a foam upon activation of the blowing agent. Instead, any bubbles that form along the face of the film or polymeric layer tend to rupture thereby creating a non-uniform, relatively discontinuous outer surface of the film. This surface is periodically referred to herein as a "cratered surface."

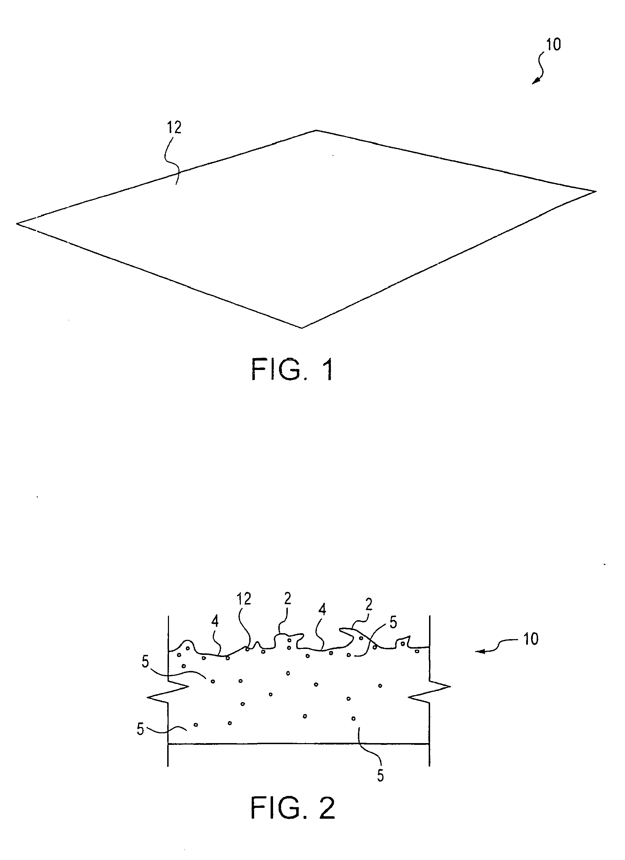

Figures 1 and 2 schematically depict a preferred embodiment film 10 defining an exposed outer face 12. Figure 2 is a representative cross section of the film 10 depicting the noted cratered surface 12. Specifically, the film 10 comprises a layer of a polymeric composition including one or more antimicrobial agents 5 dispersed throughout the layer. The antimicrobial agents 5 can for example include silver ions that are accessible along the outer cratered surface 12 of the film 10. The cratered surface 12 generally includes a plurality of outwardly extending projections 2 and a plurality of recessed depressions 4. As will be appreciated, the surface area of the outer surface 12 is significantly increased as a result of the presence of the projections and depressions 4.

As previously noted, the polymeric film may utilize an antimicrobial promoter that is a hydrophilic wetting agent. Thus, one or more hydrophilic wetting agents can be included in the polymeric composition of the film 10 depicted in Figures 1 and 2. Alternatively, the polymeric composition can use a promoter that exclusively includes hydrophilic wetting agents and thus is free of blowing agents. In these embodiments, the polymeric film exhibits a relatively smooth outer face unlike the cratered surface resulting when the promoter includes blowing agent(s). These aspects are described in greater detail herein.

### Antimicrobial Agent

Preferably, the at least one antimicrobial agent incorporated in the polymeric layer comprises an organic antimicrobial agent, an inorganic antimicrobial agent, or a mixture of two or more of any of the foregoing antimicrobial agents. The antimicrobial agent can be microbicidal and kill microbes, or can be microbistatic and prevent growth or reproduction of microbes. The term "microbes" as used herein refers to microorganisms which include bacteria, fungi, plants, and protozoans. Additional examples of microbes are included herein.

In a preferred embodiment, the organic antimicrobial agent comprises a natural antimicrobial agent to include for example phytochemicals such as citric acid, a synthetic antimicrobial agent to include for example triclosan and quaternary ammonium halides, or a mixture of two or more of any of the foregoing organic antimicrobial agents.

The inorganic antimicrobial agent preferably comprises a metal ion-containing composition. In a preferred embodiment, the inorganic antimicrobial agent comprises a silver ion-containing composition, a zinc ion-containing composition, a copper ion-containing composition, a tin ion-containing composition, or a mixture of two or more of any of the foregoing compositions. Most preferably, the inorganic antimicrobial agent is a silver-ion containing composition.

In certain embodiments the antimicrobial agent is an inorganic antimicrobial agent which is microbistatic. The metal ion-containing composition can comprise a metal ion on an organic support such as for example an ion exchange resin, a metal ion on an inorganic support such as for example a silicate based zeolite or a silicate based clay or a glass or a ceramic, or a mixture of two or more of any of the foregoing compositions. In certain embodiments the antimicrobial agent of the polymeric layer comprises an antimicrobial agent dispersed in a thermoplastic polymer carrier to form an antimicrobial agent additive concentrate. In other embodiments the antimicrobial agent of the polymeric layer comprises an antimicrobial agent functionalized nanometer-sized filler additive and the functionalization involves linking the antimicrobial agent on the surface of the filler. This functionalization can result in a smaller particle size and increased dispersion of the antimicrobial agent and greater efficiency and longevity of the antimicrobial agent compared to antimicrobial agents on a conventional micrometer-sized support or filler.

A particularly preferred antimicrobial agent is commercially available under the designation BACTIBLOCK^{®}, from NanoBioMatters North America, LLC of Boston, MA. BACTIBLOCK^{®} is an antimicrobial additive for polymer-based raw materials. The BACTIBLOCK^{®} technology is based on silver-functionalized clay that creates a naturally sourced and highly efficient antimicrobial product. The additive prevents the growth of bacteria, mold, fungus and other microorganisms, which also makes BACTIBLOCK^{®} a powerful tool against odors and stains.

The active ingredient in BACTIBLOCK^{®} is ionic silver, a naturally occurring element with a well-known antimicrobial spectrum, as well as being widely recognized as safe for human contact. In the BACTIBLOCK^{®} agent, ionic silver is linked to the clay surface, thereby providing a uniform distribution of the active species within the additive. In addition, the silver deposited on the clay prevents platelet agglomeration, which ensures uniform additive dispersion. The combination of these dispersion mechanisms maximizes antimicrobial efficiency and homogenous protection of the polymer material.

The silver species linked to the clay platelets are released to the surface of the protected material at a controlled rate. This ensures a more uniform and long term antimicrobial effect, as compared to additives with the active species readily available in the polymer.

The major component of each BACTIBLOCK^{®} grade is functionalized clay, combined with a low concentration of antimicrobial agent. The specific levels of each component can be adjusted to achieve the desired performance. The final concentration of the total additive package is highly dependent on product design and target properties.

Additional details as to various features and formation of the preferred silver-functionalized clays are set forth in US 2010/0272831.

The antimicrobial agent may also be selected from a wide range of known antibiotics and antimicrobials. Suitable materials are discussed in "Active Packaging of Food Applications" A. L. Brody, E. R. Strupinsky, and L. R. Kline, Technomic Publishing Company, Inc. Pennsylvania (2001). Additional examples of antimicrobial agents suitable for practice of the invention include benzoic acid, sorbic acid, nisin, thymol, allicin, peroxides, imazalil, triclosan, benomyl, antimicrobial metal-ion exchange material, metal colloids, metal salts, anhydrides, and organic quaternary ammonium salts.

In a preferred embodiment, the antimicrobial agent is selected from metal ion-exchange materials which have been exchanged or loaded with antimicrobial ions. Metal ion-exchange materials suitable for practice of the invention are selected from zirconium phosphates, metal hydrogen phosphates, sodium zirconium hydrogen phosphates, zeolites, clays such as montmorillonite, ion-exchange resins and polymers, porous alumino-silicates, layered ion-exchange materials, and magnesium silicates. Preferred metal ion exchange materials are zirconium phosphate, metal hydrogen phosphate, sodium zirconium hydrogen phosphate, or zeolite. Preferred antimicrobial ions are silver, copper, nickel, zinc, tin, and gold. In a particularly preferred embodiment the antimicrobial ions are selected from silver and zinc. The antimicrobial ion is the antimicrobial moiety of the antimicrobial agent.

Additional examples of preferred antimicrobial agents include, but are not limited to, chlorhexidine gluconate (CHG), benzalkonium chloride (BZK), or iodopropynylbutyl carbamate (IPBC; Germall plus). Further examples of antimicrobial agents include, but are not limited to, iodophors, iodine, benzoic acid, dihydroacetic acid, propionic acid, sorbic acid, methyl paraben, ethyl paraben, propyl paraben, butyl paraben, cetrimide, quaternary ammonium compounds, including but not limited to benzethonium chloride (BZT), dequalinium chloride, biguanides such as chlorhexidine (including free base and salts (see below)), PHMB (polyhexamethylene biguanide), chloroeresol, chlorxylenol, benzyl alcohol, bronopol, chlorbutanol, ethanol, phenoxyethanol, phenylethyl alcohol, 2,4-dichlorobenzyl alcohol, thiomersal, clindamycin, erythromycin, benzoyl peroxide, mupirocin, bacitracin, polymyxin B, neomycin, triclosan, parachlorometaxylene, foscarnet, miconazole, fluconazole, itriconazole, ketoconazole, and pharmaceutically acceptable salts thereof.

Pharmaceutically acceptable chlorhexidine salts that may be used as antimicrobial agents according to the invention include, but are not limited to, chlorhexidine palmitate, chlorhexidine diphosphanilate, chlorhexidine digluconate, chlorhexidine diacetate, chlorhexidine dihydrochloride, chlorhexidine dichloride, chlorhexidine dihydroiodide, chlorhexidine diperchlorate, chlorhexidine dinitrate, chlorhexidine sulfate, chlorhexidine sulfite, chlorhexidine thiosulfate, chlorhexidine di-acid phosphate, chlorhexidine difluorophosphate, chlorhexidine diformate, chlorhexidine dipropionate, chlorhexidine di-iodobutyrate, chlorhexidine di-n-valerate, chlorhexidine dicaproate, chlorhexidine malonate, chlorhexidine succinate, chlorhexidine malate, chlorhexidine tartrate, chlorhexidine dimonoglycolate, chlorhexidine monodiglycolate, chlorhexidine dilactate, chlorhexidine di-alpha-hydroxyisobutyrate, chlorhexidine diglucoheptonate, chlorhexidine di-isothionate, chlorhexidine dibenzoate, chlorhexidine dicinnamate, chlorhexidine dimandelate, chlorhexidine di-isophthalate, chlorhexidine di-2-hydroxynapthoate, and chlorhexidine embonate. Chlorhexidine free base is a further example of an antimicrobial agent.

The polymeric layer(s) of the present invention can also contain any combination of additional medicinal compounds. Such medicinal compounds include, but are not limited to, antimicrobials, antibiotics, antifungal agents, antiviral agents, antithrombogenic agents, anesthetics, anti-inflammatory agents, analgesics, anticancer agents, vasodilation substances, wound healing agents, angiogenic agents, angiostatic agents, immune boosting agents, growth factors, and other biological agents. Suitable antimicrobial agents include, but are not limited to, biguanide compounds, such as chlorhexidine and its salts; triclosan; penicillins; tetracyclines; aminoglycosides, such as gentamicin and Tobramycin™; polymyxins; rifampicins; bacitracins; erythromycins; vancomycins; neomycins; chloramphenicols; miconazole; quinolones, such as oxolinic acid, norfloxacin, nalidixic acid, pefloxacin, enoxacin, and ciprofloxacin; sulfonamides; nonoxynol 9; fusidic acid; cephalosporins; and combinations of such compounds and similar compounds. The additional antimicrobial compounds provide for enhanced antimicrobial activity.

The antimicrobial compound is preferably used in an effective amount. The term "effective amount" as used herein refers to any amount of the antimicrobial agent(s) such that when incorporated into a polymeric composition and film is formed therefrom, the presence of the agent along a face of the film kills or at least prevents growth or reproduction of microbes. It is preferred, when the antimicrobial ion is silver, that the silver ion comprises 0.01 to 20% by weight of the composition. Most preferably, the concentration of the silver ion in the polymeric layer is from about 5% to about 15%.

In the present invention, film surfaces are rendered resistant to microbial growth. Some of the microbes which can be resisted include single cell organisms, e.g., bacteria, fungi, algae, and yeast, and mold. The bacteria can include both gram positive and gram negative bacteria. Some examples of gram positive bacteria include, for example, bacillus cereus, micrococcus luteus, and staphylococcus aureus. Some examples of gram negative bacteria include, for example, escherichia coli, enterobacter aerogenes, enterobacter cloacae, and proteus vulgaris. Strains of yeast include, for example, saccharomyces cerevisiae. It will be appreciated that the invention is directed toward killing or reducing growth and/or reproduction of a wide array of other microbes and microorganisms. In no way is the invention limited to the particular examples of microbes presented herein.

### Blowing Agent as Antimicrobial Promoter

As previously noted, in certain embodiments, the antimicrobial promoter may include one or more blowing agents. The promoter may include such blowing agents with one or more hydrophilic wetting agents, or exclusively use the blowing agents. The blowing agent utilized in the present invention can be selected from any known blowing agent suitable for the respective polymer, for example, from aliphatic or cycloaliphatic compounds including hydrocarbons, ethers, lower alcohols, halogenated hydrocarbons, especially partially halogenated hydrocarbons, and inorganic blowing agents such as water, carbon dioxide, nitrous oxides such as NO, NO₂ and N₂O, nitrogen, ammonia, noble gases such as argon and air, or mixtures thereof. Inorganic blowing agents can also be produced in situ by adding chemical compounds to the composition which decompose and generate gas, such as known typically in the art, for example, azo-type compounds for the generation of N₂, ammonium compounds of the generation of NH₃ and mixtures of carbonates and acids for the generation of CO₂.

Examples of suitable aliphatic or cycloaliphatic compounds are ethane, ethylene, propane, propylene, butane, isobutane, butylene, isobutene, pentane, neopentane, isopentane, cyclopentane, hexane, heptane, cyclohexane and mixtures thereof. Suitable examples of ethers are dimethyl ether (DME), methyl ethyl ether, or diethyl ether. Suitable examples of lower alcohols are methanol, ethanol, propanol, isopropanol, butanol, pentanol, hexanol and mixtures thereof, wherein ethanol is preferred. Among the inorganic blowing agents, carbon dioxide or carbon dioxide/water mixtures are preferred. Examples of partially halogenated hydrocarbons are chloroethane, chlorodifluoromethane (R-22), 1-chloro-1,1-difluoroethane (R-142b), 1,1,1,2-tetrafluoroethane (R-134a), 1,1,2,2-tetrafluoroethane (R-134), 2-chloro-1,1,1,2-tetrafluoroethane (R-124), pentafluoroethane (R-125), 1,1-difluoroethane (R-152a), 1,1,1-trifluoroethane (R-143a), 1-fluoroethane (R-161), difluoromethane (R-32), 1,1,1,3,3-pentafluoropropane (HFC-245 fa), 1,1,1,3,3-pentafluorobutane (HFC-365 mfc) and mixtures thereof.

Preferable in all cases are blowing agent compositions which have no ozone depletion potential (ODP), namely fluorinated alkanes, inorganic blowing agents, alcohols, hydrocarbons, ethers or combinations thereof. Particularly suitable, for example, for alkylene aromatic polymers and copolymers, or for olefinic polymers and copolymers, are blowing agent compositions composed primarily of carbon dioxide, and mixtures of carbon dioxide with water or ethanol or isopropanol or dimethyl ether or mixtures of two or more of these. Compositions based on (i) 1,1,1,2-tetrafluoroethane, (ii) 1,1,2,2-tetrafluoroethane, (iii) 1,1-difluoroethane, (iv) mixtures of two or more of these, or (v) mixtures of each compound or mixture with ethanol or isopropanol or dimethyl ether or water or carbon dioxide or mixtures of two or more of these are also particularly suitable in the practice of the present invention. Additionally, compositions based on dimethyl ether and mixtures of dimethyl ether with water or ethanol or isopropanol or carbon dioxide or mixtures of two or more of these are also suitable in the practice of the present invention. Other suitable blowing agents are hydrocarbons, such as propane, butane, pentane or mixtures thereof. Furthermore, mixtures of suitable hydrocarbons with dimethyl ether, carbon dioxide, and partially halogenated hydrocarbons are also suitable in the practice of the present invention.

The blowing agent is generally used in an amount of from 0.5 to 2.5 weight percent, and preferably from 1 to 1.5 weight percent based on the total weight of the composition.

Preferably, the blowing agent does not disrupt the surface to the point that sealing is no longer possible. This means that the cell size, created by the blowing agent, preferably has a cross-sectional area less than the thickness of the polymeric layer containing the antimicrobial. Also, preferably the blowing agent does not interfere with the function of the antimicrobial agent. Therefore, the blowing agent does not render the surface more hydrophobic or generate a competing anionic effect that would negate the antimicrobial function. This is particularly so when the antimicrobial promoter also includes a hydrophilic wetting agent. These are preferred characteristics of the noted cratered surface of the preferred films.

Generally, when the antimicrobial promoter includes blowing agents, the size of the bubbles are not uniform, and they depend upon the amount of blowing agent and the processing temperature. The surface break will depend upon the location of the bubble relative to the surface.

A typical and preferred size range of the bubbles (or of the ruptured bubbles) is as follows. The maximum diameter in the plane of the polymeric film is preferably between 20% to 50% of the polymeric layer thickness. This is another preferred characteristic of the noted cratered surface of the preferred embodiment films. However, it will be appreciated that in no way is the invention limited to this characteristic. Instead, the invention includes polymeric films formed in which the maximum diameter of bubbles is less than 20% of the polymeric layer thickness and greater than 50% of the polymeric layer thickness.

### Hydrophilic Wetting Agent as Antimicrobial Promoter

As noted, in certain embodiments, the antimicrobial promoter includes one or more hydrophilic wetting agents. The promoter may include such wetting agents with one or more blowing agents, or exclusively use the wetting agents. The term "wetting agent" refers to an agent which facilitates or enhances the hydration or contact with an aqueous medium along a face of the polymeric layer. Examples of suitable wetting agents include polyoxyalkylenes (such as those available from BASF under the designation PLURONICS, or those available from Union Carbide under the designation CARBOWAXES, including those with PEGs), ether capped polyoxyalkylenes, e.g., polyoxyethylene lauryl ether, ester capped polyoxyalkylenes, e.g., polyoxyethylene stearate, sorbitan esters (such as certain products commercially available under the designations SPAN and TWEEN), phosphatides (such as lecithin), alkyl amines, glycerin, water soluble polymers such as polyethylene oxides, carboxymethyl cellulose, polyvinyl alcohol, and polyvinyl pyrrolidone, surfactants such as alkyl (C₆-C₂₀) sulfate salts, e.g., sodium lauryl sulfate, aryl (C₆-C₁₀) sulfate salts, and alkaryl (C₇-C₂₄) sulfate salts, and the like. It will be understood that in no way is the invention limited to any of these wetting agents. Instead, nearly any wetting agent can be used for the antimicrobial promoter so long as the wetting agent is compatible with the polymeric component, and the antimicrobial agent.

Although not wishing to be bound to any particular theory, it is believed that the hydrophilic wetting agent(s) serves as an antimicrobial agent promoter by increasing the extent of contact between a face of the polymeric film and an aqueous medium which likely contains microbes. The increased contact between the medium and the polymeric film surface increases the extent of exposure to the antimicrobial agent(s) within the film.

### Methods

The various preferred embodiment films can be produced in variety of methods. Preferably, in forming a polymeric film having antimicrobial properties, one or more antimicrobial agents and one or more antimicrobial agent promoters are selected and combined in appropriate amounts with a polymeric system as described herein. The components may be combined in a single operation or may be combined and/or incorporated into the polymeric system sequentially and in multiple operations. Films are then formed from the polymeric composition. Preferably, known extrusion or co-extrusion techniques are used to form the films from one or more polymeric compositions or polymer components and additive components. When utilizing blowing agent(s) for the antimicrobial promoter, appropriate conditions such as temperature and extrusion rate are selected so as to produce a desired cratered surface.

The present invention also provides various methods. A preferred method involves increasing the effectiveness of an antimicrobial agent incorporated within a polymeric composition. The method includes an operation of adding an antimicrobial agent promoter as described herein to the composition. This method can be used to increase the effectiveness of films formed from the composition, and of the compositions themselves.

### Multilayer Barrier

The present invention also provides multilayer films for use in medical applications and in particular, for ostomy applications. As will be appreciated, an important characteristic for such films is preventing or at least significantly reducing transmission of odors through the film. The preferred films feature a polymeric layer comprising an antimicrobial agent in combination with an antimicrobial agent promoter. The agent(s) and promoter(s) are as previously described herein. A preferred multilayer barrier construction uses odor blocking layers that include a cyclic olefin copolymer (COC) in optional combination with a compatibilizer. In other embodiments, the preferred multilayer barrier construction includes an inner layer containing nanoclay particles. In yet additional embodiments, the multilayer barrier construction can utilize these components together in separate or common layers or regions of the multilayer assembly. In still additional embodiments, an odor barrier composite is utilized in the multilayer barrier film. The odor barrier composite includes layers of a norbornene-based material which is preferably cyclic olefin copolymer or COC and/or ethylene vinyl alcohol (EVOH). Preferably, the odor barrier composite includes one or more layer(s) having a norbornene-based material and one or more other components, and one or more additional layer(s) having EVOH. Preferably, at least one of the layers containing the norbornene-based material is immediately adjacent to the layer containing EVOH. In a particularly preferred embodiment, a layer containing one or more grades of EVOH is disposed or "sandwiched" between two layers each of which includes the norbornene-based materials.

In certain embodiments, it is preferred to utilize one or more cyclic olefin copolymers in the polymeric film. Cyclic olefin copolymers are also known as ethylene copolymer, COC, cyclo olefinecopolymer, cyclic olefin polymer, and ethylene-norbornene copolymer.

Presently, there exist several types of commercially available cyclic olefin copolymers based on different types of cyclic monomers and polymerization methods. Cyclic olefin copolymers are typically produced by chain copolymerization of cyclic monomers such as 8,9,10-trinorborn-2-ene (norbornene) or 1,2,3,4,4a,5,8,8a-octahydro-1,4:5,8-dimethanonaphthalene (tetracyclododecene) with ethene. Non-limiting examples of commercially available cyclic olefin copolymers include those available from TOPAS Advanced Polymers under the designation TOPAS, Mitsui Chemical's APEL, or those formed by ring-opening metathesis polymerization of various cyclic monomers followed by hydrogenation, which are available from Japan Synthetic Rubber under the designation ARTON, and Zeon Chemical's ZEONEX and ZEONOR.

Particularly preferred cyclic olefin copolymers are those available from TOPAS Advanced Polymers. These cyclic olefin copolymers, in contrast to the partially crystalline polyolefins polyethylene and polypropylene, are generally amorphous, transparent copolymers based on cyclic olefins and linear olefins.

Most preferably, the cyclic olefin copolymers from TOPAS are grades 9506 and 8007. Another preferred cyclic olefin copolymer from TOPAS is also noted below. This cyclic olefin copolymer is referred to as a developmental polymer. The properties and characteristics of these copolymers are set forth below in Tables 1-3.

**Table 1- Grade 9506 From TOPAS**

| Property | Value | Unit | Test Standard |
|---|---|---|---|
| Physical Properties | | | |
| Density | 1020 | kg/m³ | ISO 1183 |
| Melt volume rate (MVR) | 6.0 | cm³/10 min | ISO 1133 |
| MVR test temperature | 230 | °C | ISO 1133 |
| MVR test load | 2.16 | kg | ISO 1133 |
| Melt volume rate (MVR) - 2nd value | 1.0 | cm³/10 min | ISO 1133 |
| MVR test temperature - 2nd value | 190 | °C | ISO 1133 |
| MVR test load - 2nd value | 2.16 | kg | ISO 1133 |
| Water absorption (23°C-sat) | 0.01 | % | ISO 62 |

| Thermal Properties | | | |
|---|---|---|---|
| Glass transition temperature 10°C/min | 65 | °C | ISO 11357-1,-2,-3 |

| Mechanical Properties (Film) | | | |
|---|---|---|---|
| Tensile modulus (machine direction) | 1700 | MPa | ISO 527-3 |
| Tensile modulus (transverse direction | 2000 | MPa | ISO 527-3 |
| Tensile strength @ break (machine direction) | 55 | MPa | ISO 527-3 |
| Tensile strength @ break (transverse direction) | 55 | MPa | ISO 527-3 |
| Elongation at break (machine direction) | 2.9 | % | ISO 527-3 |
| Elongation at break (transverse direction) | 3.6 | % | ISO 527-3 |
| Elmendorf tear strength (machine direction) | 230 | g | ISO 6383-2 |
| Elmendorf tear strength (transverse direction) | 240 | g | ISO 6383-2 |
| Dart Drop Impact Strength, F50 | <36 | g | ISO 7765-1 |

| Optical Properties (Film) | | | |
|---|---|---|---|
| Gloss, 60° | >100 | % | ISO 2813 |
| Haze | <1 | % | ISO 14782 |

| Barrier Properties (Film) | | | |
|---|---|---|---|
| Water vapor permeability @ 38°C, 90% RH | 0.8 | g·100µm/(m²·day) | ISO 15106-3 |
| Oxygen Permeability @ 23°C, 50% RH | 170.0 | cm³·100µm/(m²·day·bar) | ASTM D3985 |

**Table 2 - Grade 8007F-04 From TOPAS**

| Property | | Value | Unit | Test Standard |
|---|---|---|---|---|
| Physical Properties | | | | |
| Density | | 1020 | kg/m³ | ISO 1183 |
| Melt volume rate (MVR) | | 12.0 | cm³/10 min | ISO 1133 |
| | MVR test temperature | 230 | °C | ISO 1133 |
| | MVR test load | 2.16 | kg | ISO 1133 |
| Melt volume rate (MVR) - 2nd value | | 2.0 | cm³/10 min | ISO 1133 |
| | MVR test temperature - 2nd value | 190 | °C | ISO 1133 |
| | MVR test load - 2nd value | 2.16 | kg | ISO 1133 |
| Water absorption (23°C-sat) | | 0.01 | % | ISO 62 |

| Thermal Properties | | | | |
|---|---|---|---|---|
| Glass transition temperature 10°C/min | | 78 | °C | ISO 11357-1,-2,-3 |

| Mechanical Properties (Film) | | | | |
|---|---|---|---|---|
| Tensile modulus (machine direction) | | 2200 | MPa | ISO 527-3 |
| Tensile modulus (transverse direction) | | 1800 | MPa | ISO 527-3 |
| Tensile strength @ break (machine direction) | | 57 | MPa | ISO 527-3 |
| Tensile strength @ break (transverse direction) | | 50 | MPa | ISO 527-3 |
| Elongation at break (machine direction) | | 2.9 | % | ISO 527-3 |
| Elongation at break (transverse direction) | | 3.0 | % | ISO 527-3 |
| Elmendorf tear strength (machine direction) | | 225 | g | ISO 6383-2 |
| Elmendorf tear strength (transverse direction) | | 230 | g | ISO 6383-2 |
| Dart Drop Impact Strength, F50 | | <36 | g | ISO 7765-1 |

| Optical Properties (Film) | | | | |
|---|---|---|---|---|
| Gloss, 60° | | >100 | % | ISO 2813 |
| Haze | | <1 | % | ISO 14782 |

| Barrier Properties (Film) | | | | |
|---|---|---|---|---|
| Water vapor permeability @ 38°C, 90% RH | | 0.8 | g·100µm/(m²·day) | ISO 15106-3 |
| Oxygen Permeability @ 23°C, 50% RH | | 200.0 | cm³·100µm/(m²·day·bar) | ASTM D3985 |

**Table 3 - Developmental Cyclic Olefin Copolymer From TOPAS**

| Property | Value | Unit | Test Standard |
|---|---|---|---|
| Physical Properties | | | |
| Density | 0.940 | g/cm³ | ISO 1183 |
| Melt volume rate (MVR) at 190°C and 2.16 kg | 3.0 | cm³/10 min | ISO 1133 |
| Melt volume rate (MVR) at 260°C and 2.16 kg | 12.0 | cm³/10 min | ISO 1133 |
| Hardness, shore A | 89 | - | ISO 868 |
| WVTR (23°C / 85% RH) | 1.0 | g*100µm/m² *day | ISO 15106-3 |
| WVTR (38°C / 90% RH) | 4.6 | g*100µm/m²*day | ISO 15106-3 |
| OTR (23°C / 50% RH) | 1060 | cc*100µm/m2 *day | ISO 15105-2 |
| | | | |

| Mechanical Properties | | | |
|---|---|---|---|
| Tensile stress at break (50mm/min) | >19 | MPa | ISO 527-T2/1A |
| Tensile modulus (1mm/min) | 44 | MPa | ISO 527-T2/1A |
| Tensile strain at break (50mm/min) | >450 | % | ISO 527-T2/1A |
| Compression set (24hr / 23°C) | 35 | % | ISO 815 |
| Compression set (72hr / 23°C) | 32 | % | ISO 815 |
| Compression set (24hr / 60°C) | 90 | % | ISO 815 |
| Tear strength | 47 | kN/m | ISO 34-1 |
| Puncture resistance | 44 | J | ASTM D3786-08 |
| | | | |

| Film Properties - 50 µm / 2 mil cast film | | | |
|---|---|---|---|
| Tensile stress at break (machine direction) | 8.8 | ksi | ASTM D882 |
| Tensile modulus (MD) | 5.26 | ksi | ASTM D882 |
| Elongation at break (MD) | 640 | % | ASTM D882 |
| Tensile stress at break (transverse direction) | 7.31 | ksi | ASTM D882 |
| Tensile modulus (TD) | 4.93 | ksi | ASTM D882 |
| Elongation at break (TD) | 620 | % | ASTM D882 |
| Elmendorf tear (MD, 3200 grams) | 1103 | grams | ASTM D1922 |
| Elmendorf tear (TD, 3200 grams) | 1221 | grams | ASTM D1922 |
| Haze | 1.5 | % | ASTM D1003 |
| Gloss (60°C) | 94 | % | ASTM D2457 |
| | | | |

| Electrical Properties | | | |
|---|---|---|---|
| Relative permittivity at 1 GHz | 2.24 | - | ASTM D2520 / B |
| Dissipation factor at 1 GHz | 2.6E-04 | - | ASTM D2520 / B |
| Dielectric strength | 4000 | V/mil | ASTM D149-97a |
| | | | |

| Thermal Properties | | | |
|---|---|---|---|
| Ductile-brittle temperature | <-90 | °C | ISO 974 |
| Tm - melt temperature | 84 | °C | Internal method |
| Vicat softening temperature, VST/A50 | 64 | °C | ISO 306 |

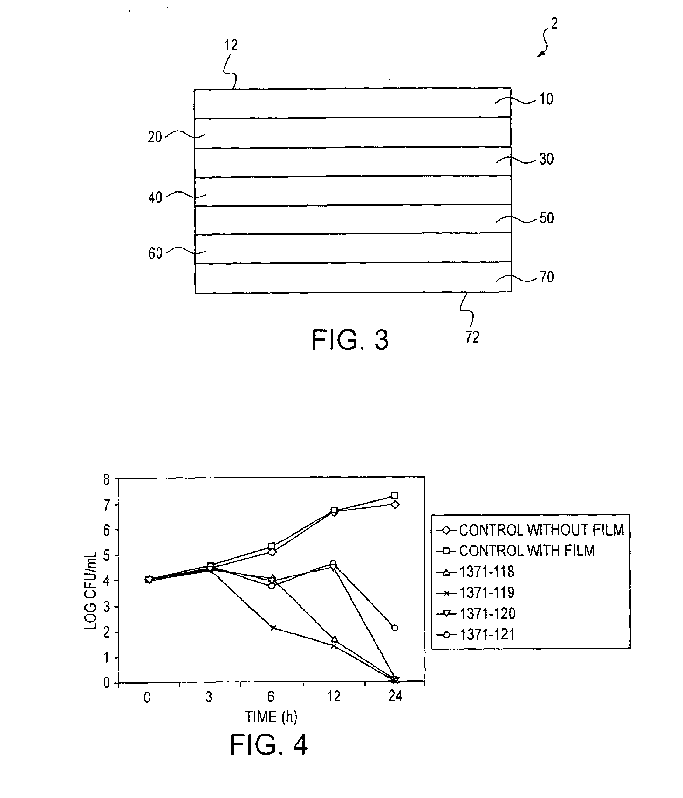

Figure 3 is a schematic illustration of a preferred multilayer barrier assembly 2 in accordance with the present invention. The multilayer assembly 2 comprises an outer layer 10 defining an outer face 12, a first flexible support layer 10, a first moisture and odor barrier layer(s) 30, a secondary barrier 40 for reducing transmission of oxygen and odors, a second moisture and odor barrier 50, a second flexible support layer 60, and an inner antimicrobial layer 70 defining an inner face 72 for contacting a microbe containing medium. The antimicrobial layer 70 includes at least one antimicrobial agent and at least one antimicrobial agent promoter as described herein. In the event that the antimicrobial agent promoter includes one or more blowing agents, it is preferred that the inner face 72 of the multilayered barrier assembly exhibits a cratered surface as described herein.

Generally, the preferred embodiment multilayer constructions include (the layers are noted in order from the outermost layer to the innermost layer): (i) an outer nonwoven layer, (ii) a first flexible support for dampening noise using an elastomer of polyethylene and polypropylene, (iii) a first moisture and odor barrier layer or layer assembly including the COC and a compatibilizer, g-maleic anhydride EVA, (iv) another barrier for reducing transmission of oxygen and hydrogen sulfide (i.e. odor) that includes EVOH and a nanoclay component to provide a tortuous path to further reduce the potential for transfer of chemical agents or species across the barrier, (v) a second moisture and odor barrier corresponding to previously noted layer (iii), (vi) a second flexible support corresponding to previously noted layer (ii), and (vii) an inner antimicrobial layer containing an antimicrobial agent such as a silver ion containing compound.

A preferred multilayer barrier construction is set forth in Table 4 as follows:

**Table 4 - Preferred Multilayer Film**

| Layer | Weight Percent | Layer Description |
|---|---|---|
| (i) | 5% | 90% EVA (18% VA) |
| | | 10% Slip/AB |
| (ii) | 35% | 100% Polyolefin elastomer |
| (iii) | 5% | 70% Cyclic Olefin Copolymer (COC) |
| | | 30% GMAH-EVA tie |
| (iv) | 10% | 47% EVOH (48 mol) |
| | | 35% EVOH nanoclay |
| | | 18% E/MAA lonomer (Zn neutralized) |
| (v) | 5% | 70% Cyclic Olefin Copolymer (COC) |
| | | 30% GMAH-EVA tie |
| (vi) | 35% | 100% Polyolefin elastomer |
| (vii) | 5% | 45% EVA (28% VA) |
| | | 30% Antimicrobial masterbatch (10% active) |
| | | 11% Metallocene-Catalyzed LLDPE |
| | | 10% Slip/AB |
| | | 4% Hydrophilic Wetting Agent |

The COC preferred for use in the multilayer film construction, e.g. in layers (iii) and (v), is obtained from TOPAS Advanced Polymers. The COC is believed to be a copolymer of norbornene and ethylene. At present, Grade 9506 from TOPAS is preferred for use in the various multilayer barrier constructions. It is also contemplated that Grade 8007 or the previously noted developmental COC may be used. The properties and characteristics of these copolymers are set forth in Tables 1-3 herein. The COC can be used in the moisture or odor barrier layer in nearly any concentration, such as from about 10% to about 95%, more preferably from about 50% to about 90%, more preferably from about 60% to about 80%, and most preferably about 70%. In certain embodiments, it is preferred to use the COC in combination with a tie component such as g-maleic anhydride ethylene vinyl acetate (GMAH-EVA). It will be appreciated that the various multilayer barrier films are not limited to the use of these particular COC's. Instead, it is contemplated that a wide range of comparable compounds and/or materials could be utilized in the noted moisture and odor barrier layer(s) of the multilayer construction.

The flexible supports, e.g. layers (ii) and (vi), in the preferred multilayer construction utilize a low density polyolefin and preferably, a propylene and/or ethylene based elastomer. It will be appreciated that the present invention multilayer barrier constructions are not limited to the use of these particular elastomers.

A wide array of commercially available polyolefin elastomers can be used for one or both of the flexible support layers. Representative preferred examples of such materials include KRATON D1164P and G2832 available from Kraton Polymers US, LLC of Houston, TX; DOW AFFINITY DG8200 and DOW VERSIFY 3200 and 3000 from Dow Chemical Corp. of Midland, MI; DYNAFLEX G2755 from GLS Corp. of McHenry, IL; SEPTON 2063 from Kuraray of Tokyo, Japan; and VISTAMAXX VM1100 from Exxon Mobil Chemical Co. of Houston, TX. Table 5 set forth below presents representative modulus, tear strength, and density values for films made using these materials.

**Table 5 - Summary of Modulus, Tear Strength and Density**

| Core Resin Name | Modulus [MPa] | | Tear Strength [g] | | Density [g/cm³] |
|---|---|---|---|---|---|
| | MD | TD | MD | TD | |
| KRATON D1164P | 40.3 | 9.6 | 95 | 776 | 0.96 |
| KRATON G2832 | 5.0 | 1.7 | 179 | 207 | 1.01 |
| DOW AFFINITY DG8200 | 8.8 | 8.1 | 150 | 188 | 0.93 |
| DYNAFLEX G2755 | 9.8 | 3.2 | 175 | 186 | 0.89 |
| DOW VERSIFY 3200 | 52.8 | 59.3 | 368 | 1026 | 0.93 |
| DOW VERSIFY 3000 | 128.9 | 138.4 | 554 | 1205 | 0.87 |
| Kuraray SEPTON 2063 | 8.0 | 8.1 | 83 | 56 | 1.00 |
| Exxon VISTAMAXX VM1100 | 7.1 | 7.6 | 108 | 111 | 0.98 |

It will be appreciated that the present invention multilayer barrier constructions are not limited to the use of these particular elastomers.

The antimicrobial layer, e.g. layer (vii), preferably comprises an antimicrobial agent and one or more antimicrobial agent promoters as described herein. In this particular example, the antimicrobial agent promoter is a hydrophilic wetting agent. The antimicrobial layer also preferably comprises one or more sealable polymers as described herein such as metallocene-catalyzed linear low density polyethylene (LLDPE) and ethylene vinyl acetate (EVA). One or more slip and/or antiblocking agents can also be incorporated into this layer.

As stated previously, in addition to barrier properties, it is often desirable that a polymeric film not emit noise when crumpled. For example, in ostomy or incontinence applications, it is desirable that the ostomy or incontinence bag not emit noise. However, when crumpled, most polymeric films emit noise. In the case of the preferred embodiment multilayer barrier films, the films are significantly quieter than comparable ostomy films.

The preferred multilayer barrier construction is believed to exhibit several advantages over currently known ostomy films. The preferred multilayer barrier is halogen-free and avoids the use of polyvinylidene chloride (PVDC). The preferred multilayer barrier is quieter and exhibits significantly less "rustle". And, the preferred multilayer barrier exhibits superior odor blocking characteristics. The multilayer barrier construction may be transparent or contain coloring agents. In addition, the preferred multilayer barrier features an antimicrobial layer that exhibits a relatively high antimicrobial efficacy at least in part due to the antimicrobial agent promoter.

Another group of preferred embodiments relate to multilayered polymeric films that include particular skin layers and core and tie layers. Specifically, an embodiment of the present invention is a multilayered polymeric film that comprises a first skin layer comprising at least one gas and vapor barrier organic polymer and having an upper surface and a lower surface, a core layer comprising at least one elastomeric organic polymer and having an upper surface and a lower surface where the upper surface of the core layer underlies the lower surface of the first skin layer, and a second skin layer comprising at least one sealable organic polymer and at least one antimicrobial agent and having an upper surface and a lower surface where the upper surface of the second skin layer underlies the lower surface of the core layer. The terms "underlies" and "overlies" mean that when a first layer underlies or overlies a second layer that the first layer can partially or fully cover the second layer and that the first layer can be in direct contact with the second layer or one or more intermediate layers, to include for example tie layers, can be located between the first layer and second layer. In another embodiment of the invention the multilayered polymeric film, as described herein, further comprises at least one tie layer comprising at least one adhesive organic polymer and having an upper surface and a lower surface where the upper surface of the tie layer underlies the lower surface of the first skin layer and the lower surface of the tie layer overlies the upper surface of the core layer or the upper surface of the tie layer underlies the lower surface of the core layer and the lower surface of the tie layer overlies the upper surface of the second skin layer. In yet another embodiment of the invention an article comprises the multilayered polymeric film as described herein. The inventive multilayered polymeric film and articles derived from this film provide a combination of features that can lead to improved performance in various applications as described in greater detail hereinbelow. The features provided include a gas and vapor barrier, antimicrobial activity, a relatively low tensile modulus, and a relatively high tear strength. The inventive film and articles derived from the film are useful in a number of applications that comprise medical and health care, building and construction, sporting goods, electronics, personal care, food markets, and textiles. Medical and health care applications comprise ostomy pouches, urine bags, ulcer or wound dressings and related materials, and dialysis materials used in treatment of kidney or renal failure where treatments include hemodialysis and peritoneal dialysis and hemofiltration. The inventive film is especially useful in derived articles involving microbial activity in which the combined features can provide in the article flexibility, softness, quietness, and strength with odor control and antimicrobial activity which can, when the article is for example an ostomy pouch, reduce or eliminate venting of built up gases and vapors.

### First Skin Layer

In an embodiment of the invention the first skin layer comprises at least one gas and vapor barrier organic polymer. Gases can be defined as elements and compounds which exist in the gaseous state at standard temperature of 0°C and standard pressure of 1 atmosphere. Gases include oxygen, nitrogen, carbon dioxide, methane, and hydrogen sulfide. Vapors can be defined as a gas or an air dispersion of molecules of a substance that is liquid or solid in its normal state at standard temperature of 0°C and standard pressure of 1 atmosphere. Vapors include water, indoles, thiols such as methanethiol, and sulfides such as dimethyl disulfide. In applications involving microbial activity such as for example in ostomy pouches, malodorous gases and vapors can be formed from microbial metabolism to include indoles, thiols, sulfides and hydrogen sulfide. The first skin layer, which comprises at least one gas and vapor barrier organic polymer, can provide a gas and vapor barrier.

In an embodiment of the invention the gas and vapor barrier organic polymer of the first skin layer comprises a homopolymer or copolymer of an unsaturated chlorine-containing monomer to include the monomer vinylidene chloride, a polyamide, a vinyl alcohol containing homopolymer or copolymer to include poly(vinyl alcohols) and alkene-vinyl alcohol copolymers, a homopolymer or copolymer of acrylonitrile, a homopolymer or copolymer of styrene, a polyester, a polymethacrylate, a polycarbonate, or a mixture of two or more of any of the foregoing gas and vapor barrier organic polymers.

In an embodiment of the invention the gas and vapor barrier organic polymer of the first skin layer comprises an alkene-vinyl alcohol copolymer where the alkene monomer includes at least one C₂ to C₁₀ alpha-olefin and the alkene monomer content in the alkene-vinyl alcohol copolymer on a mole basis is 20 to 60%, 29 to 56%, or 38 to 52%. In an embodiment the gas and vapor barrier organic polymer of the first skin layer comprises a modified alkene-vinyl alcohol copolymer that contains a first alkene comonomer, a second vinyl alcohol comonomer usually derived from vinyl acetate which is hydrolyzed to form the vinyl alcohol after the copolymer is polymerized, and a third comonomer. In an embodiment the third comonomer can be present in the modified alkene-vinyl alcohol copolymer on a mole basis up to 10%, 7%, or 4%. In an embodiment the third comonomer comprises a polymerizable unsaturated monomer to include alpha-olefins such as for example ethylene or propylene or 1-butene, and unsaturated carboxylic acids or derivatives thereof such as for example acrylic acid or methyl methacrylate. The modified alkene-vinyl alcohol copolymer functions as a gas and vapor barrier organic polymer where incorporation of the third comonomer provides higher flexibility, softness and quietness in the first skin layer and the inventive film. In an embodiment of the invention the gas and vapor barrier organic polymer of the first skin layer comprises an ethylene-vinyl alcohol copolymer (EVOH). In an embodiment the gas and vapor barrier organic polymer of the first skin layer comprises a modified ethylene-vinyl alcohol copolymer as described above. In an embodiment the gas and vapor barrier organic polymer content of the first skin layer on a weight basis is greater than 50%, 55%, 60%, or 65%. In an embodiment the gas and vapor barrier organic polymer content in the first skin layer on a weight basis is 51 to 100%, 56 to 88%, or 61 to 77%. Useful gas and vapor barrier organic polymers are commercially available and include Kuraray ethylene-vinyl alcohol copolymers EVAL^{®} H101 which has an ASTM D 792 density of 1.17 g/cm³ and an ASTM D 1238 melt index at 190°C/2.16 kg of 1.6 dg/minute, EVAL^{®} E105 which has an ASTM D 792 density of 1.14 g/cm³ and an ASTM D 1238 melt index at 190°C/2.16 kg of 5.5 dg/minute, and EVALCA^{®} G176 which has an ASTM D 792 density of 1.12 g/cm³ and an ASTM D 1238 melt index at 210°C/2.16 kg of 15 dg/minute; and Nippon Gohsei ethylene-vinyl alcohol copolymers SOARNOL^{®} DC3212 which has an ASTM D 792 density of 1.19 g/cm³ and an ASTM D 1238 melt index at 210°C/2.16 kg of 12 dg/minute, and SOARNOL^{®} H4815B which has an ASTM D 792 density of 1.12 g/cm³ and an ASTM D 1238 melt index at 210°C/2.16 kg of 16 dg/minute.

In an embodiment of this invention the first skin layer further comprises at least one alkene-unsaturated carboxylic acid or unsaturated carboxylic acid derivative copolymer. The alkene-unsaturated carboxylic acid or unsaturated carboxylic acid derivative copolymer can function to improve flexibility, softness and quietness in the first skin layer and the inventive film. In an embodiment the alkene comonomer of the alkene-unsaturated carboxylic acid or unsaturated carboxylic acid derivative copolymer comprises C₂ to C₁₀ alpha-olefins. In an embodiment the unsaturated carboxylic acid comonomer of the alkene-unsaturated carboxylic acid or unsaturated carboxylic acid derivative copolymer comprises polymerizable unsaturated carboxylic acid monomers such as for example acrylic acid, methacrylic acid, and fumaric acid. In an embodiment the unsaturated carboxylic acid derivative comonomer of the alkene-unsaturated carboxylic acid or unsaturated carboxylic acid derivative copolymer comprises polymerizable unsaturated carboxylic acid derivative monomers to include esters or anhydrides such as for example vinyl acetate, methyl acrylate, ethyl methacrylate, and maleic anhydride. In an embodiment the alkene-unsaturated carboxylic acid or unsaturated carboxylic acid derivative copolymer comprises an ionomer. In an embodiment the ionomer comprises partially neutralized metal salts of alkene-unsaturated carboxylic acid copolymers where the alkene comonomer comprises C₂ to C₁₀ alpha-olefins, the unsaturated carboxylic acid comonomer comprises acrylic acid or methacrylic acid, and the metal comprises zinc, sodium, lithium, magnesium, or a mixture of two or more of the foregoing metals. In an embodiment the ionomer includes a partially neutralized zinc, sodium, or zinc and sodium salt of an ethylene-methacrylic acid copolymer. In an embodiment the alkene-unsaturated carboxylic acid or unsaturated carboxylic acid derivative copolymer comprises a mixture of two or more of the copolymers. In an embodiment the content of the alkene-unsaturated carboxylic acid or unsaturated carboxylic acid derivative copolymer in the first skin layer on a weight basis is 0 to 40%, 8 to 35%, or 16 to 30%. Useful alkene-unsaturated carboxylic acid or unsaturated carboxylic acid derivative copolymers are commercially available and include ionomers SURLYN^{®} 1705-1 from DuPont™ which has an ASTM D 792 density of 0.95 g/cm³ and an ASTM D 1238 melt index at 190°C/2.16 kg of 5.5 dg/minute, and CLARIX^{®} 210805-01 from A. Schulman^{®} which has an ASTM D 792 density of 0.94 g/cm³ and an ASTM D 1238 melt index at 190°C/2.16 kg of 4.5 dg/minute.

In an embodiment of the invention the first skin layer further comprises at least one additive to improve the performance of the first skin layer and the inventive film. Although additives can be introduced as is into one or more layers of the inventive film, they are usually introduced as part of an additive concentrate into one or more layers of the inventive film or alternatively one or more additives can be present in commercially available thermoplastic polymers that are obtained from thermoplastic polymer manufacturers to form the layers of the inventive film. The additive concentrate generally comprises at least one additive and at least one thermoplastic polymer carrier where the additive content in the additive concentrate on a weight basis can be 0.01 to 90%. In an embodiment one or more additive concentrates are present in the first skin layer on a weight basis from 0 to 30%. In an embodiment the additive of the first skin layer comprises an antiblock agent, a slip agent, a process aid, a filler, a nucleating agent, an antistatic agent, a colorant or pigment, a dye, a cavitating agent, an antioxidant, a deodorant as described hereinbelow for the second skin layer, an antimicrobial agent as described hereinbelow for the second skin layer, a plasticizer, an ultraviolet radiation absorber, or a mixture of two or more of any of the foregoing additives. In an embodiment the first skin layer comprises a slip agent, an antiblock agent, a process aid, a filler, or a mixture of two or more of any of the foregoing additives. Antiblock agents include inorganic materials, such as for example an amorphous silica, or organic compounds, such as for example a polymer which is incompatible with the polymer or polymers used to form the film layer, where the antiblock agent can serve to roughen a surface so that a film does not stick to itself. Slip agents include waxy organic materials such as for example fatty acid amides that reduce the coefficient of friction of the film. Process aids include organic lubricating materials such as for example fluoropolymers that improve flow of thermoplastic polymers during processing to form a film or layers of a film. Fillers comprise inorganic particulate solid materials such as for example clays, organic particulate solid materials, or mixtures thereof. The fillers can have a mean particle size equal to or less than 25 micrometers and can function to improve performance including mechanical, thermal and gas/vapor barrier properties in the first skin layer and in the inventive film. In an embodiment the filler comprises a nanometer-sized filler, as described in European Patent Application Publication No. EP1985585A1, where the nanometer-sized filler comprises a filler having dimensions which in at least one of the dimensions is of the order of one to tens of nanometers. In an embodiment the filler of the nanometer-sized filler comprises minerals having sheet-like layers to include phyllosilicates, such as for example clays, layered double hydroxides, or mixtures of two or more of any of the foregoing minerals. In an embodiment the nanometer-sized filler is dispersed by intercalation in a polymer to form a nanometer-sized filler in polymer additive concentrate. In an embodiment the polymer in the nanometer-sized filler additive concentrate comprises any thermoplastic polymer to include a gas and vapor barrier organic polymer as described hereinabove, an alkene-unsaturated carboxylic acid or unsaturated carboxylic acid derivative copolymer as described hereinabove, or a mixture of two or more of any of the foregoing thermoplastic polymers. In an embodiment the polymer in the nanometer-sized filler additive concentrate is an ethylene-vinyl alcohol copolymer. The nanometer-sized filler can provide improved performance relative to a conventional micrometer-sized filler. Useful additive concentrates are commercially available and include combined slip/antiblock agent additive concentrates CONPOL^{®} 5B10S1 from DuPont^{™} which has an ASTM D 792 density of 0.96 g/cm³ and an ASTM D 1238 melt index at 190°C/2.16 kg of 25 g/10 minutes, and POLYBATCH^{®} MCE 5106 IM from A. Schulman^{®} which has an ASTM D 792 density of 0.97 g/cm³; process aid additive concentrate Ampacet™ 10919 which has an ASTM D 792 density of 0.91 g/cm³ and an ASTM D 1238 melt index at 190°C/2.16 kg of 2 g/10 minutes; and a nanometer-sized filler in ethylene-vinyl alcohol copolymer additive concentrate NANOBIOTER^{®} from NanoBioMatters™.

Table 6 lists examples of compositions, on a weight percent basis, which are useful to form the first skin layer of the inventive film.

**Table 6 - First Skin Layer Examples**

| Component Identity | Example, Weight % | | |
|---|---|---|---|
| | 2 | 3 | 4 |
| Nippon Gohsei EVOH, SOARNOL^{®} DC3212 | 70 | | |
| Kuraray EVOH, EVAL^{®} H101 | | 62 | |
| Kuraray EVOH, EVALCA^{®} G176 | | | 67 |
| A. Schulman^{®} ionomer, CLARIX^{®} 210805-01 | 21 | | |
| DuPont™ ionomer, SURLYN^{®} 1705-1 | | 31 | 25 |
| DuPont™ slip/antiblock concentrate, CONPOL^{®} 5B10S1 | 8 | 6 | 7 |
| Ampacet™ process aid concentrate, 10919 | 1 | 1 | 1 |

### Core Layer

The core layer, in an embodiment of this invention, comprises at least one elastomeric organic polymer. The elastomeric organic polymer can provide both flexibility with quietness and strength to the core layer and the inventive film. In an embodiment the elastomeric organic polymer is elastic and has an ASTM D 882 elongation at break of greater than 200%, 400%, or 600%. In an embodiment the elastomeric organic polymer provides flexibility and has an ASTM D 882 tensile modulus in both the machine direction and transverse direction of less than 40 MPa (mega Pascals), 33 MPa, 26 MPa, or 19 MPa. The machine direction is the direction that the film is advanced during its manufacture, and the transverse direction is the direction that is normal or perpendicular to the machine direction. In an embodiment the elastomeric organic polymer provides strength and has an ASTM D 882 tensile strength at break in both the machine direction and transverse direction of at least 10 MPa, 11 MPa, 12 MPa, or 13 MPa.

In an embodiment of the invention the elastomeric organic polymer of the core layer comprises a vinyl arene-based block copolymer elastomer, a polyurethane-based elastomer, a polyester-based elastomer, a polyamide-based elastomer, a polyolefin-based elastomer, or a mixture of two or more of any of the foregoing elastomers. In an embodiment the vinyl arene-based block copolymer elastomer comprises a copolymer of a vinyl arene, to include vinyl arenes such as for example styrene and alpha-methylstyrene, with at least one other monomer to form two or more polymer blocks within the copolymer. In an embodiment the other monomer comprises at least one olefin where the olefin comprises an alkene which can be an alpha-olefin where the alkene or alpha-olefin has two or more carbon atoms, a nonconjugated diene, a conjugated diene, or a mixture of two or more of any of the foregoing olefins. In an embodiment olefins include ethylene, propylene, isobutylene, 2-butene, 1,4-pentadiene, isoprene, butadiene, or a mixture of two or more of any of the foregoing olefins. In an embodiment vinyl arene-based block copolymer elastomers comprise a styrene-isoprene diblock copolymer which can be hydrogenated or nonhydrogenated, a styrene-isoprene-styrene triblock copolymer which can be hydrogenated or nonhydrogenated, a styrene-ethylene/propylene-styrene triblock copolymer, a styrene-ethylene/butylene-styrene triblock copolymer, a styrene-butadiene diblock copolymer which can be hydrogenated or nonhydrogenated, a styrene-butadiene-styrene triblock copolymer which can be hydrogenated or nonhydrogenated, a styrene-isoprene-butadiene block copolymer which can be hydrogenated or nonhydrogenated, or a mixture of two or more of any of the foregoing block copolymers to include mixtures of styrene-isoprene-styrene triblock copolymers and styrene-isoprene diblock copolymers (SIS/SI). In an embodiment the vinyl arene-based block copolymer elastomer has a low vinyl arene content which results in increased elasticity. In an embodiment the vinyl arene-based block copolymer elastomer has a vinyl arene content on a mole basis of less than 50%, 40%, or 30%. In an embodiment the vinyl arene-based block copolymer elastomer has a vinyl arene content on a weight basis of less than 50%, 40%, or 30%. In an embodiment the vinyl arene-based block copolymer elastomer includes a linear block copolymer, a branched block copolymer, a radial block copolymer, or a combination of two or more of any of the foregoing block copolymers.

In embodiments the polyolefin-based elastomer comprises at least one polymer formed from one olefin, as described hereinabove for vinyl arene-based block copolymer elastomers, or at least one polymer formed from one olefin where the polymer is a block copolymer, for example, a block copolymer having at least one isotactic or syndiotactic polypropylene block and at least one atactic polypropylene block. In an embodiment the polyolefin-based elastomer comprises at least one copolymer formed from two or more olefins, as described hereinabove for vinyl arene-based block copolymer elastomers, and comprising a random copolymer, a block copolymer having at least two polymer blocks, or a mixture of two or more of any of the foregoing copolymers. In an embodiment the polyolefin-based elastomer random copolymer comprises a random copolymer formed from at least a first alkene and at least a second alkene or diene where the content of the second alkene or diene in the random copolymer on a mole basis is 25% or less to include, for example, a linear low density polyethylene having a density of greater than 0.912 up to 0.94 g/cm³ and formed from a major amount of ethylene and a minor amount of at least one other alpha-olefin, and a butyl rubber formed from a major amount of isobutylene and a minor amount of isoprene; a random copolymer formed from at least two alkenes where the two alkenes are generally present in the random copolymer in equimolar amounts to include, for example, an ethylene-propylene rubber, and an ethylene-propylene-diene monomer elastomer which additionally contains a minor amount of a nonconjugated diene; or a mixture of two or more of any of the foregoing random copolymers. In an embodiment the polyolefin-based elastomer block copolymer comprises a block copolymer formed from at least two alkenes and having at least one homopolymer segment bonded to at least one copolymer segment to include, for example, a propylene-ethylene-based block copolymer containing at least one polypropylene homopolymer segment and at least one propylene-ethylene copolymer segment, and an ethylene-alpha-olefin-based block copolymer containing at least one polyethylene homopolymer segment and at least one ethylene-alpha-olefin copolymer segment; a block copolymer formed from at least one linear diene and at least one branched diene and having at least one homopolymer segment from the linear diene bonded to at least one homopolymer segment from the branched diene, to include, for example, a hydrogenated butadiene-isoprene-butadiene triblock copolymer; or a mixture of two or more of any of the foregoing block copolymers. In an embodiment the polyolefin-based elastomer comprises a block copolymer formed from at least two alkenes, as described hereinabove, where the block copolymer has its crystalline size in the nanometer level which provides improved properties compared to conventional elastomers which have their crystalline size in the micrometer level. Polyolefin-based elastomer block copolymers are further described in US Patent Nos. 3,985,826 and 5,708,083 and 6,812,292. In an embodiment the core layer comprises a blend of an elastomeric organic polymer and at least one second organic polymer or component where the content of the elastomeric organic polymer in the core layer on a weight basis is greater than 50%, 70%, or 90%. In an embodiment the content of the elastomeric organic polymer in the core layer on a weight basis is greater than 50 up to 100%, greater than 70 up to 100%, or greater than 90 up to 100%. Useful elastomeric organic polymers are commercially available and include a polyolefin-based elastomer NOTIO™ PN-2070 (a propylene-ethylene block copolymer with crystalline size in the nanometer level) from Mitsui Chemicals which has an ASTM D 792 density of 0.867 g/cm³, an ASTM D 1238 melt flow rate at 230°C/2.16 kg of 7 dg/minute, an ASTM D 882 elongation at break of greater than 800%, an ASTM D 882 tensile modulus in machine and transverse directions of 16 MPa, an ASTM D 882 tensile strength at break in machine and transverse directions of 14 MPa; a polyolefin-based elastomer VISTAMAXX™ VM1100 (a propylene-ethylene-based random copolymer) from ExxonMobil which has an ASTM D 792 density of 0.862 g/cm³ and an ASTM D 1238 melt flow rate at 230°C/2.16 kg of 4 dg/minute; and a vinyl arene-based block copolymer elastomer VECROR™ 4114A (a SIS/SI blend of block copolymers) from Dexco Polymers which has an ASTM D 792 density of 0.92 g/cm³ and an ASTM D 1238 melt flow rate at 200°C/5 kg of 25 dg/minute

In an embodiment of the invention the core layer further comprises at least one additive, as described hereinabove for the first skin layer, to improve the performance of the core layer and the inventive film. In an embodiment the core layer comprises at least one colorant. In an embodiment one or more additive concentrates are present in the core layer on a weight basis from 0 to 30%. Useful additive concentrates are commercially available and include a colorant additive concentrate Ampacet™ Beige 6027E.

Table 7 lists examples of compositions, on a weight percent basis, which are useful to form the core layer of the inventive film.

**Table 7 - Core Layer Examples**

| Component Identity | Example, Weight % | | |
|---|---|---|---|
| | 2 | 3 | 4 |
| Mitsui Chemicals polyolefin elastomer, NOTIO™ PN-2070 | 100 | | |
| ExxonMobil polyolefin elastomer, VISTAMAXX™ VM1100 | | 100 | |
| Dexco Polymers vinyl arene block copolymer elastomer SIS/SI blend, VECTOR™ 4114A | | | 100 |

### Second Skin Layer

In an embodiment of this invention the second skin layer comprises at least one sealable organic polymer and at least one antimicrobial agent. In an embodiment the sealable organic polymer comprises any thermoplastic organic polymer that allows sealing of the second skin layer to itself or another layer of the inventive film, to a layer of a different film, or to an article. In embodiments of the invention the sealing method includes sealing with an adhesive such as for example a pressure sensitive adhesive or solvent adhesive, sealing via a lamination using a laminate adhesive and/or pressure and/or heat, sealing with heat, or any combination of the foregoing sealing methods. In an embodiment the sealing method is a heat sealing method. In an embodiment the heat sealing method comprises sealing with a heated bar, sealing with hot air, sealing with an electromagnetic radiation source such as for example infrared radiation or radio frequency radiation, or a combination of any of the foregoing heat sealing methods. In an embodiment the sealable organic polymer comprises a polyolefin, a polyester, a polyamide, a styrene-based homopolymer or copolymer, a polycarbonate, a polyurethane, a halogen-containing polymer, an acrylate-based or methacrylate-based homopolymer or copolymer, an alkene-unsaturated carboxylic acid or unsaturated carboxylic acid derivative copolymer as described for the first skin layer and hereinbelow, a vinyl alcohol-containing homopolymer or copolymer, or a mixture of two or more of any of the foregoing polymers. In an embodiment of the invention the sealable organic polymer is heat sealable and comprises a polyethylene, an alkene-unsaturated carboxylic acid or unsaturated carboxylic acid derivative copolymer, or a mixture of two or more of any of the foregoing polymers. In an embodiment the polyethylene is heat sealable and comprises a high density polyethylene, a medium density polyethylene, a low density polyethylene, a linear low density polyethylene, a very low density polyethylene, a polyethylene plastomer, or a mixture of two or more of any of the foregoing polyethylenes. In an embodiment the alkene-unsaturated carboxylic acid or unsaturated carboxylic acid derivative copolymer is heat sealable and comprises an ethylene-vinyl acetate copolymer, an ethylene-methacrylic acid copolymer, an ethylene-acrylic acid copolymer, an ethylene-alkyl methacrylate copolymer, an ethylene-alkyl acrylate copolymer, an ionomer to include metal salts of ethylene-methacrylic acid copolymers, or a mixture of two or more of any of the foregoing copolymers. In an embodiment the ethylene-vinyl acetate copolymer is heat sealable and on a weight basis has a vinyl acetate content of 10 to 40%, 16 to 36%, or 22 to 32%. In an embodiment the sealable organic polymer is present on a weight basis in the second skin layer at 51 to 99.99%, 65 to 99.99%, or 80 to 99%. Useful sealable organic polymers are commercially available and include ethylene-vinyl acetate (EVA) copolymers ATEVA^{®} 2810A from Celanese which has an ASTM D 792 density of 0.949 g/cm³ and an ASTM D 1238 melt index at 190°C/2.16 kg of 6 dg/minute and a vinyl acetate content of 28% by weight, and ELVAX^{®} 3182 from DuPont™ which has an ASTM D 792 density of 0.95 g/cm³ and an ASTM D 1238 melt index at 190°C/2.16 kg of 3 dg/minute and a vinyl acetate content of 28% by weight.

In an embodiment of the invention the at least one antimicrobial agent of the second skin layer comprises an organic antimicrobial agent, an inorganic antimicrobial agent, or a mixture of two or more of any of the foregoing antimicrobial agents. The antimicrobial agent can be microbicidal and kill microbes, or can be microbistatic and prevent growth or reproduction of microbes. Microbes are microorganisms which include bacteria, fungi, and protozoans. In an embodiment the organic antimicrobial agent comprises a natural antimicrobial agent to include for example phytochemicals such as citric acid, a synthetic antimicrobial agent to include for example triclosan and quaternary ammonium halides, or a mixture of two or more of any of the foregoing organic antimicrobial agents. In an embodiment the inorganic antimicrobial agent comprises a metal ion-containing composition. In an embodiment the inorganic antimicrobial agent comprises a silver ion-containing composition, a zinc ion-containing composition, a copper ion-containing composition, a tin ion-containing composition, or a mixture of two or more of any of the foregoing compositions. In an embodiment the antimicrobial agent is an inorganic antimicrobial agent which is microbistatic. In an embodiment the metal ion-containing composition comprises a metal ion on an organic support such as for example an ion exchange resin, a metal ion on an inorganic support such as for example a silicate based zeolite or a silicate based clay or a glass or a ceramic, or a mixture of two or more of any of the foregoing compositions. In an embodiment the at least one antimicrobial agent of the second skin layer comprises an antimicrobial agent dispersed in a thermoplastic polymer carrier to form an antimicrobial agent additive concentrate. In an embodiment the at least one antimicrobial agent of the second skin layer comprises an antimicrobial agent functionalized nanometer-sized filler additive where the nanometer-sized filler is described hereinabove regarding the first skin layer and the functionalization involves linking the antimicrobial agent on the surface of the filler. This functionalization can result in a smaller particle size and increased dispersion of the antimicrobial agent and greater efficiency and longevity of the antimicrobial agent compared to antimicrobial agents on a conventional micrometer-sized support or filler. Additionally the nanometer-sized filler, as described hereinabove regarding the first skin layer, can further improve performance relative to conventional micrometer-sized fillers. In an embodiment the antimicrobial agent functionalized nanometer-sized filler additive comprises an inorganic antimicrobial agent comprising a metal ion-containing composition to include a silver ion-containing composition, a zinc ion-containing composition, a copper ion-containing composition, a tin ion-containing composition, or a mixture of two or more of any of the foregoing compositions. In an embodiment the at least one antimicrobial agent of the second skin layer, to include an antimicrobial agent functionalized nanometer-sized filler additive, is dispersed in a thermoplastic polymer carrier to form an antimicrobial agent additive concentrate. In an embodiment the thermoplastic polymer carrier of the antimicrobial agent additive concentrate comprises any thermoplastic polymer to include a polyolefin, a polyester, a polyamide, a styrene-based homopolymer or copolymer, a polycarbonate, a polyurethane, a halogen-containing polymer, an acrylate-based or methacrylate-based homopolymer or copolymer, an alkene-unsaturated carboxylic acid or unsaturated carboxylic acid derivative copolymer, a vinyl alcohol-containing homopolymer or copolymer to include for example a poly(vinyl alcohol), or a mixture of two or more of any of the foregoing polymers. In an embodiment the thermoplastic polymer carrier of the antimicrobial agent additive concentrate comprises a sealable organic polymer that is heat sealable, as described hereinabove, to include a polyethylene, an alkene-unsaturated carboxylic acid or unsaturated carboxylic acid derivative copolymer, or a mixture of two or more of any of the foregoing polymers. In an embodiment the antimicrobial agent of the second skin layer is delivered to the second skin layer in an additive concentrate where the additive concentrate comprises the antimicrobial agent and a poly(vinyl alcohol) carrier. In an embodiment the antimicrobial agent additive concentrate comprises an antimicrobial agent or an antimicrobial agent on a support or filler dispersed in a thermoplastic polymer carrier where the content on a weight basis of the antimicrobial agent or the antimicrobial agent on the support or filler is 0.01 to 90%. In an embodiment the content of one or more antimicrobial agent additive concentrates in the second skin layer on a weight basis is 0.01 to 30%. Useful antimicrobial agents are commercially available and include silver ion based antimicrobial agent additive concentrates POLYBATCH^{®} ABACT 422 VA from A. Schulman^{®}, and BACTIGLAS^{®} AM 91874 from Wells Plastics; silver based antimicrobial agent additive NANOBIOTER+^{®} from NanoBioMatters which is a silver functionalized nanometer-sized clay filler additive that can be supplied as an additive concentrate in specified polymer carriers; zinc oxide based antimicrobial agent additive concentrate ANTIBACTERIAL-PE^{®} CC10098866 from PolyOne; triclosan based antimicrobial agent additive concentrate AMPE^{®} 115283 CC10115283 from PolyOne; and antimicrobial agent additive concentrate MCX^{®} 117653 from BIOSAFE^{®}.

In an embodiment of the invention the second skin layer further comprises at least one additional additive, as described hereinabove for the first skin layer, to improve the performance of the second skin layer and the inventive film. In an embodiment the second skin layer further comprises an antiblock agent, a slip agent, a filler, a deodorant, or a mixture of two or more of any of the foregoing additives. In an embodiment the deodorant comprises any material that absorbs odor to include a beta-cyclodextrin, an organic acid, a metal salt of a fatty carboxylic acid such as for example a zinc ricinoleate, an aluminum compound such as for example an alumina, a silicon compound such as for example a silica, an iron compound such as for example ferrous sulfate, a zinc compound such as for example a zinc silicate, a clay or clay derivative such as for example a zeolite, or a mixture of two or more of any of the foregoing deodorants. In an embodiment one or more additive concentrates, containing at least one additional additive, are present in the second skin layer on a weight basis from 0 to 30%. Useful additional additives are commercially available and include a combined slip agent/antiblock agent additive concentrate POLYBATCH^{®} 1220 NG from A. Schulman^{®}, a deodorant additive CAVAMAX^{®} W7 from Wacker Chemical which is a beta-cyclodextrin, and a deodorant additive concentrate Ampacet^{®} 101787 which contains an aluminum oxide.

Table 8 lists examples of compositions, on a weight percent basis, which are useful to form the second skin layer of the inventive film.

**Table 8 - Second Skin Layer Examples**

| Component Identity | Example, Weight % | | |
|---|---|---|---|
| | 2 | 3 | 4 |
| DuPont™ EVA, ELVAX^{®} 3182 | 80 | | 87 |
| Celanese EVA, ATEVA^{®} 2810A | | 84 | |
| A. Schulman^{®} slip/antiblock concentrate, POLYBATCH^{®} 1220NG | 10 | 10 | 9 |
| PolyOne zinc antimicrobial concentrate, ANTIBACTERIAL-PE^{®} CC10098866 | 10 | | |
| A. Schulman^{®} silver antimicrobial concentrate, POLYBATCH^{®} ABACT 422 VA | | 6 | 4 |

### Tie Layer

In an embodiment the inventive film further comprises at least one tie layer where the tie layer comprises at least one adhesive organic polymer. The adhesive organic polymer of the tie layer or tie layers can provide increased bonding or adhesion between one or both of the skin layers and the core layer to prevent delamination while maintaining flexibility. In embodiments the inventive film has two tie layers where the two tie layers have the same composition, or where the two tie layers have different compositions. In an embodiment the adhesive organic polymer of a tie layer comprises any thermoplastic organic polymer that provides increased bonding or adhesion between the first or second skin layer and the core layer. In an embodiment the adhesive organic polymer comprises an alkene-unsaturated carboxylic acid or unsaturated carboxylic acid derivative copolymer as described for the first and second skin layers, a polyethylene as described for the second skin layer, an alkene-unsaturated carboxylic acid or unsaturated carboxylic acid derivative copolymer grafted with an unsaturated carboxylic acid or unsaturated carboxylic acid derivative grafting component, a polyolefin grafted with an unsaturated carboxylic acid or unsaturated carboxylic acid derivative grafting component, or a mixture of two or more of any of the foregoing adhesive organic polymers. In an embodiment the unsaturated carboxylic acid or unsaturated carboxylic acid derivative grafting component comprises an unsaturated carboxylic acid having three or more carbon atoms, an unsaturated carboxylic acid derivative to include an ester or an anhydride of an unsaturated carboxylic acid having three or more carbon atoms, or a mixture of two or more of any of the foregoing grafting components. The unsaturated carboxylic acid or unsaturated carboxylic acid derivative grafting component includes acrylic acid, methacrylic acid, vinyl acetate, glycidyl methacrylate, maleic anhydride, or a mixture of two or more of any of the foregoing grafting components. In an embodiment the polyolefin grafted with an unsaturated carboxylic acid or unsaturated carboxylic acid derivative grafting component comprises a polyolefin homopolymer to include polyethylene or polypropylene homopolymers, a polyolefin copolymer to include polyethylene or polypropylene copolymers, or a mixture of two or more of any of the foregoing polyolefins. In an embodiment the adhesive organic polymer of a tie layer comprises an ethylene-vinyl acetate copolymer that has been grafted with an unsaturated carboxylic acid or unsaturated carboxylic acid derivative grafting component. Useful adhesive organic polymers are commercially available and include the maleic anhydride grafted ethylene-vinyl acetate copolymer BYNEL^{®} E418 from DuPont™ which has an ASTM D 792 density of 0.95 g/cm³ and an ASTM D 1238 melt index at 190°C/2.16 kg of 10.9 dg/minute. In an embodiment the tie layer further comprises at least one additive as described hereinabove for the first skin layer.

### Film Construction, Processing, and Articles and Methods Thereof

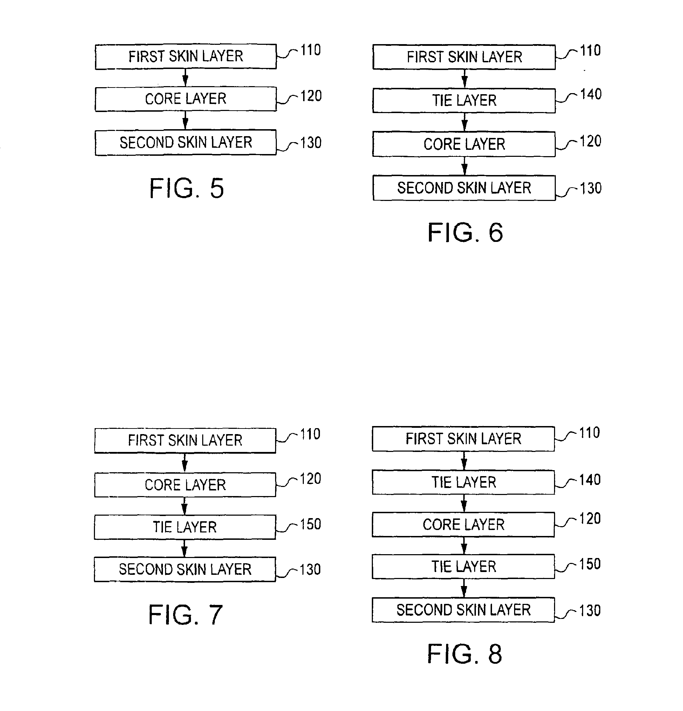

In an embodiment, as depicted in Figure 5, the inventive multilayered polymeric film comprises a first skin layer 110 having an upper surface and a lower surface, a core layer 120 having an upper surface and a lower surface where the upper surface of the core layer underlies the lower surface of the first skin layer, and a second skin layer 130 having an upper surface and a lower surface where the upper surface of the second skin layer underlies the lower surface of the core layer. In an embodiment, as depicted in Figure 6, the inventive multilayered polymeric film comprises a first skin layer 110 having an upper surface and a lower surface, a core layer 120 having an upper surface and a lower surface, a tie layer 140 having an upper surface and a lower surface where the upper surface of the tie layer underlies the lower surface of the first skin layer and the lower surface of the tie layer overlies the upper surface of the core layer, and a second skin layer 130 having an upper surface and a lower surface where the upper surface of the second skin layer underlies the lower surface of the core layer. In an embodiment, as depicted in Figure 7, the inventive multilayered polymeric film comprises a first skin layer 110 having an upper surface and a lower surface, a core layer 120 having an upper surface and a lower surface where the upper surface of the core layer underlies the lower surface of the first skin layer, a second skin layer 130 having an upper surface and a lower surface, and a tie layer 150 having an upper surface and a lower surface where the upper surface of the tie layer underlies the lower surface of the core layer and the lower surface of the tie layer overlies the upper surface of the second skin layer. In an embodiment, as depicted in Figure 8, the inventive multilayered polymeric film comprises a first skin layer 110 having an upper surface and a lower surface, a core layer 120 having an upper surface and a lower surface, a tie layer 140 having an upper surface and a lower surface where the upper surface of the tie layer 140 underlies the lower surface of the first skin layer and the lower surface of the tie layer 140 overlies the upper surface of the core layer, a second skin layer 130 having an upper surface and a lower surface, and a tie layer 150 having an upper surface and a lower surface where the upper surface of the tie layer 150 underlies the lower surface of the core layer and the lower surface of the tie layer 150 overlies the upper surface of the second skin layer.

Embodiments of the inventive multilayered polymeric film, as depicted in Figures 5-8, can be formed by one or more steps comprising extrusion of one layer from a linear or annular die to form an extrudate of one layer, coextrusion of two or more layers from a linear or annular die to form a coextrudate of two or more layers, coating of one or more layers onto a previously formed layer or multilayer composite, lamination of two or more previously formed layers, or any combination of the foregoing steps. In an embodiment the inventive film is formed by a coextrusion of two or more layers of the film. In an embodiment the inventive film is an oriented film by stretching it monoaxially, is an oriented film by stretching it biaxially, or is a nonoriented film or caste film where the film is not intentionally stretched monoaxially or biaxially.

The inventive multilayered polymeric film and the layers of the film can vary in thickness depending on the particular application of film use. In an embodiment the film has a thickness in micrometers of 13 to 254, 36 to 198, or 58 to 142. The layers of the film can vary in thickness relative to one another depending on the specific function of each layer and the particular application of film use. In an embodiment the core layer, which provides tear strength to the film, is relatively thicker than the first and second skin layers and, if present in the film, any tie layer or tie layers. In an embodiment the thickness of the core layer is 70 to 90%, 73 to 87%, or 76 to 84% of the thickness of the film. In an embodiment the thickness of the core layer in micrometers is 9 to 229, 26 to 172, or 44 to 119. In an embodiment the thickness of each of the skin layers and the tie layer or tie layers is 1 to 20%, 2 to 17%, or 3 to 14% of the thickness of the film. In an embodiment the thickness of each of the skin layers and the tie layer or tie layers in micrometers is 0.2 to 51, 0.7 to 34, or 1.7 to 20.

The multilayered polymeric film of this invention, as detailed in the Examples section, has a combination of performance properties which makes the film useful in various applications, to include medical and health care applications, especially where microbial activity is involved. In an embodiment the film has a combination of flexibility and quietness, strength, gas and vapor barrier properties, antimicrobial activity with consequent reduction in odor and odor control and reduction in gas and vapor generation due to microbial activity, and is free of chlorine or is essentially chlorine free. In an embodiment the film has flexibility and quietness, or noise reduction, as measured by the tensile modulus where a film with a lower tensile modulus is more flexible and quieter than a film with a higher tensile modulus. In an embodiment the film has an ASTM D 882 tensile modulus in both the machine direction and the transverse direction of less than 138 MPa, 108 MPa, or 78 MPa. In an embodiment the film has strength or toughness as measured by tear strength. In an embodiment the film has an ASTM D 1922 tear strength in grams force of at least 100, 164, or 228 in both the machine direction and the transverse direction. In an embodiment the film functions as a gas barrier where the film has an ASTM F 1927 oxygen transmission rate measured in cm³/(m²-day-atm) at 37°C and 90% relative humidity of less than 400, 367, or 334. In an embodiment the functions as a vapor barrier where the film has an ASTM F 1249 moisture vapor transmission rate measured in gf(m²-day-atm) at 37°C and 100% relative humidity of less than 30, 27, or 24. In an embodiment the film, which contains an antimicrobial agent, provides antimicrobial activity and consequent reduction in odor and odor control and reduction in gas and vapor generation due to microbial activity. In an embodiment the film is free of chlorine, or is essentially chlorine free and has a chlorine content on a weight basis of less than 1%, 0.7%, or 0.4%.

In an embodiment an article comprises the film of this invention. The inventive film and articles comprising the inventive film are useful in various applications as described hereinabove, especially those that require or benefit from a gas and vapor barrier and antimicrobial activity. In an embodiment an article comprises the film of this invention where the article is used in a medical or health care application. In an embodiment the article, which is used in a medical or health care application, comprises an ostomy pouch, a urine bag, an ulcer dressing, or a dialysis material used for disease treatment. In an embodiment the external or outer surface of an article for use in a medical or health care application, for example an ostomy pouch, comprises the first skin layer of the inventive film and the internal or inner surface of the article comprises the second skin layer of the inventive film.

In an embodiment of this invention a method to provide a gas and vapor barrier and antimicrobial activity in an article manufactured from a film comprises a) providing a film where the film comprises a first skin layer comprising at least one gas and vapor barrier organic polymer and having an upper surface and a lower surface, a core layer comprising at least one elastomeric organic polymer and having an upper surface and a lower surface where the upper surface of the core layer underlies the lower surface of the first skin layer, and a second skin layer comprising at least one sealable polymer and at least one antimicrobial agent and having an upper surface and a lower surface where the upper surface of the second skin layer underlies the lower surface of the core layer, and b) manufacturing an article where the article comprises the film. In an embodiment a method to provide odor control and a reduction in gases and vapors due to microbial activity in an article manufactured from a film comprises a) providing a film where the film comprises a first skin layer comprising at least one gas and vapor barrier organic polymer and having an upper surface and a lower surface, a core layer comprising at least one elastomeric organic polymer and having an upper surface and a lower surface where the upper surface of the core layer underlies the lower surface of the first skin layer, and a second skin layer comprising at least one sealable polymer and at least one antimicrobial agent and having an upper surface and a lower surface where the upper surface of the second skin layer underlies the lower surface of the core layer, and b) manufacturing an article where the article comprises the film. The inventive film combines a first skin layer having a passive gas and vapor barrier property with a second skin layer having an active antimicrobial agent. The inventive film can control odors both passively in the first skin layer due to the barrier property and actively in the second skin layer due to the antimicrobial activity of the antimicrobial agent. Additionally the inventive film can reduce gases and vapors generated from microbial activity due to the antimicrobial activity of the antimicrobial agent in the second skin layer. Articles comprising the inventive film can benefit from the combination of odor control and a reduction in gases and vapors due to microbial activity to include ostomy pouches where the need to vent built up gases and vapors can be reduced or eliminated.

### Examples

### Example 1

The following trials were conducted to further illustrate the antimicrobial properties of the preferred embodiment films comprising (i) one or more antimicrobial agents, and (ii) one or more antimicrobial agent promoters.

12 kg of an antimicrobial masterbatch were produced by obtaining and combining EVA available from ATEVA^{®} under the designation 2810A and 9.63% of BACTIBLOCK^{®} 101 R1.41. Select amounts of the antimicrobial additive BACTIBLOCK^{®} were added to various polymeric compositions containing the antimicrobial promoters described herein. Control compositions were also prepared as described in greater detail below. Polymeric film samples were formed from the compositions. The effect of the samples on microbes was then evaluated.

The concentration of the antimicrobial agent was 2.5% (a preliminary analysis of the obtained material showed an R value of 5.39 for this particular load according to JIS Z 2801). Subsequently, 7 different films were obtained for the evaluation of their antimicrobial effectiveness. The samples consisted of multilayer films of approximately 100 µm in thickness with one active side with modified EVA (ca. 10 µm). From these samples, only two films showed significant biocide properties against the growth of E. coli at the conditions employed, possibly due to the low load of antimicrobial product.

Five additional different films were then prepared and evaluated for their antimicrobial effectiveness, as set forth below in Table 9.

**Table 9 - Samples Tested for Antimicrobial Effectiveness**

| 1 | Control |
|---|---|
| 2 | 1371-118 |
| | EVOH SIDE - 48 mol % EVOH + NBM (35%) - 10% layer target |
| | Reverse Side - EVA + NBM Ag Ion (30%) - 5% layer target |
| 3 | 1371-119 |
| | EVOH SIDE - 48 mol % EVOH + NBM (35%) - 10% layer target |
| | Reverse Side - EVA + NBM Ag Ion (30%) + Surface Additive #1 - 5% layer target |
| 4 | 1371-120 |
| | EVOH SIDE - 48 mol % EVOH + NBM (35%) - 10% layer target |
| | Reverse Side - EVA + NBM Ag Ion (30%) + Surface Additive #2 - 15% layer target |
| 5 | 1371-121 |
| | EVOH SIDE - 48 mol % EVOH + NBM (35%) - 10% layer target |
| | Reverse Side - EVA + NBM Ag Ion (30%) + Surface Additive #1&2 - 15% layer target |

To test the effectiveness of the antimicrobial polymer films, the bacterial growth was evaluated using a standard test method for determining antimicrobial activity of immobilized antimicrobial agents under dynamic contact conditions (ASTM E2149).

According to this methodology, film specimens of 1.5 g were introduced in test tubes containing 10 mL of phosphate buffer solution at pH 7.4. Subsequently, tubes were inoculated with 10³ cells/mL of escherichia coli (CECT 516) in mid-exponential phase and incubated in wrist action shaker (160 rpm) at 25°C for 24h. Bacterial counts were enumerated at different internal times (3, 6, 12 and 24 h) by sub-cultivation on TSA plates.

Figure 4 shows the bacterial counts of the samples at different incubation times (0, 3, 6, 12, 24). Each value represents the mean of two replicate tubes. Figure 4 illustrates antimicrobial effectiveness of the studied films on the growth of E. coli for 24 h at 25°C and 160 rpm. From this figure, an optimal growth of the microorganism can be observed in control samples without film and control samples with the nonmodified film, achieving bacterial concentrations close to 7 log units at the end of the study. On the other hand, samples with the antimicrobial agent showed significant antimicrobial effects.

Table 10 shows the percent of microbial reduction compared to the test tubes with the nonmodified film, for each sample and interval time.

**Table 10 - Percent Microbial Reduction**

| Samples | Percent (%) Reduction | | | |
|---|---|---|---|---|
| | 3 h | 6h | 12 h | 24 h |
| 1371-118 | 16.62 | 93.35 | 100.00 | 100.00 |
| 1371-119 | 25.67 | 99.93 | 100.00 | 100.00 |
| 1371-120 | 8.61 | 95.30 | 99.33 | 100.00 |
| 1371-121 | 0.00 | 97.25 | 99.13 | 100.00 |

Specifically, the percent of microbial reduction was calculated by the following formula: % Reduction = (CFU/mL control-CFU/mL sample)/CFU/mL control *100.

As seen in Table 10, microbial reductions higher than 90% were obtained for all samples from 6 hours of incubation. Although differences between samples were very slight, the film 1371-119 appeared to provide better antimicrobial properties from the beginning of the test.

All the tested samples showed significant biocide properties against the growth of E. coli at the conditions employed.

In Examples 2-4, various multilayer films having skin layers, core layers, and/or tie layers were evaluated.

### Example 2

An 81 micrometer thick, four-layered film was prepared by coextrusion through a linear die to form the film which was a nonoriented film. The film construction was as shown in Figure 6. The first skin layer had a thickness that was 8% of the film thickness, and was formed from first skin layer Example 4 of Table 6. The tie layer had a thickness that was 8% of the film thickness, and contained on a weight basis 100% of maleic anhydride grafted ethylene-vinyl acetate copolymer DuPont™ BYNEL^{®} E418. The core layer had a thickness that was 79% of the film thickness, and was formed from core layer Example 2 of Table 7. The second skin layer had a thickness that was 5% of the film thickness, and was formed from second skin layer Example 3 of Table 8.

### Film Example 3 (comparative)

The film of comparative Example 3 was a commercial film that had a thickness of 84 micrometers. The film of Example 3 had a construction that consisted of two outer skin layers on either side of an inner core layer. Each of the two skin layers contained an ethylene-vinyl acetate copolymer, and the core layer contained a poly(vinylidene chloride).

Table 11 lists the physical properties of Film Examples 2 and 3.

**Table 11 - Physical Properties**

| Physical Property | Film Example 2 | Film Example 3 |
|---|---|---|
| Tensile Modulus¹, MPa | 47/30 | 358/168 |
| Tear Strength², grams force | 293/320 | 67/80 |
| OTR³, cm³/(m2-day-atm) | 300 | 120 |
| MVTR⁴, g/(m²-day-atm) | 20.5 | 8.2 |

| | | |
|---|---|---|
| ¹ ASTM D 882 tensile modulus measured in the machine/transverse directions. ² ASTM D 1922 tear strength measured in the machine/transverse directions. ³ ASTM F 1927 oxygen transmission rate (OTR) measured at 37°C and 90% relative humidity. ⁴ ASTM F 1249 moisture vapor transmission rate (MVTR) measured at 37°C and 100% relative humidity. | | |

Tables 12 and 13 present antimicrobial activity data for the film of this invention.

**Table 12 - Antimicrobial Data**

| Film Sample | Surface Antimicrobial Activity, JIS Z 2801³ | | |
|---|---|---|---|
| | Number of Living Bacteria | | % Reduction |
| | Initial | After 24 hrs | |
| Antimicrobial Film¹ | 1.6x10⁵ | <1x10² | >99.999 |
| Control Film² | 1.6x10⁵ | 2.1x10⁷ | ---- |

| | | | |
|---|---|---|---|
| ¹ The antimicrobial film sample contained 6% by weight of an A. Schulman silver ion-containing antimicrobial agent additive concentrate. ² The control film did not contain an antimicrobial agent. ³ Surface antimicrobial activity was measured by JIS (Japanese Industrial Standards) Z 2801 test method for Escherichia coli (NBRC 3972). Activity was based on the number of living bacteria remaining after 24 hours from an initial bacterial culture with efficacy expressed as the percent reduction. | | | |

**Table 13 - Antimicrobial Data**

| Film Sample | Dynamic Antimicrobial Activity, ASTM E 2149-01³ | | |
|---|---|---|---|
| | % Reduction from Control | | |
| | 30 min | 60 min | 120 min |
| 6% Antimicrobial Concentrate¹ | 45 | 0.0 | 17 |
| 9% Antimicrobial Concentrate² | 54 | 19 | 42 |

| | | | |
|---|---|---|---|
| ¹ The film sample had a second skin layer that contained 6% by weight of an A. Schulman silver ion-containing antimicrobial agent additive concentrate. ² The film sample had a second skin layer that contained 9% by weight of an A. Schulman silver ion-containing antimicrobial agent additive concentrate. ³ Antimicrobial activity under dynamic contact conditions was measured by ASTM E 2149-01 test method for Klebsiella pneumoniae (ATCC # 4352). Activity was based on the percent reduction in bacteria compared to a control sample after contact times of 30, 60 and 120 minutes. | | | |

Table 14 presents odor control and gas accumulation performance evaluations for articles made from Film Examples 2 and 3.

**Table 14 - Performance Evaluations**

| Performance Evaluation | Pouch from Film Example 21 | Pouch from Film Example 31 |
|---|---|---|
| Immediate Odor 2 | 1 | 3 |
| 72 Hour Odor2 | 2 | 1 |
| 72 Hour Gas Accumulation3 | 3 | 5 |

| | | |
|---|---|---|
| ¹ Equidimensional pouches were made from Film Examples 2 (which contained an antimicrobial agent) and 3 (which did not contain an antimicrobial agent), and evaluated for odor control and gas accumulation performance. Each pouch was sealed with a milk composition of 2 grams of a landscape gardening mulch and 60 ml of a pasteurized milk, and then heated at 40°C for 24 hours. ² The pouches were evaluated for odor by a committee immediately after the heating and 72 hours after the heating on a scale of 0 (no odor) to 5 (extreme odor). ³ The pouches were evaluated for gas accumulation by a committee 72 hours after the heating on a scale of 0 (no swelling of pouch) to 5 (extreme swelling of pouch). In a parallel evaluation equidimensional pouches were made from Film Examples 2 and 3 that contained 20 grams of chopped onion which generates gases/vapors due to enzymatic activity, not microbial activity. Both of the onion containing pouches, from Film Examples 2 and 3, had a rating of 5 for gas accumulation after 72 hours. | | |

The invention relates to a medical device such as a wound dressing or an ostomy appliance, an incontinence device, or other device such as a catheter; which medical device comprises a stabilized composition having antibacterial, antiviral and/or antifungal activity. The ostomy appliance of the invention may be an ostomy bag or a body side member or any ostomy device such as a closure comprising a composition according to the invention.

Many other benefits will no doubt become apparent from future application and development of this technology.

All patents, published applications, and articles noted herein are hereby incorporated by reference in their entirety.

It will be understood that any one or more feature or component of one embodiment described herein can be combined with one or more other features or components of another embodiment. Thus, the present invention includes any and all combinations of components or features of the embodiments described herein.

As described hereinabove, the present invention solves many problems associated with previously known compositions, films, and practices. However, it will be appreciated that various changes in the details, materials and arrangements of components and operations, which have been herein described and illustrated in order to explain the nature of the invention, may be made by those skilled in the art without departing from the principle and scope of the invention, as expressed in the appended claims.
The present invention also relates to the following items:
1. A polymeric composition adapted for forming a film, the composition comprising:
   at least one thermoplastic polymer;
   an antimicrobial agent; and
   an antimicrobial agent promoter selected from the group consisting of (i) at least one blowing agent, (ii) at least one hydrophilic wetting agent, and (iii) combinations of (i) and (ii).
2. The polymeric composition of item 1 wherein the thermoplastic polymer is a sealable organic polymer.
3. The polymeric composition of item 2 wherein the sealable organic polymer is ethylene vinyl acetate (EVA).
4. The polymeric composition of item 3 wherein the ethylene vinyl acetate has a vinyl acetate content of 10% to 40%.
5. The polymeric composition of item 3 wherein the ethylene vinyl acetate has a vinyl acetate content of 16% to 36%.
6. The polymeric composition of item 3 wherein the ethylene vinyl acetate has a vinyl acetate content of 22% to 32%.
7. The polymeric composition of items 1-6 further comprising linear low density polyethylene.
8. The polymeric composition of items 1-7 wherein the polymeric composition comprises from about 40% to about 50% ethylene vinyl acetate.
9. The polymeric composition of item 8 wherein the polymeric composition comprises about 45% ethylene vinyl acetate.
10. The polymeric composition of item 7 wherein the polymeric composition comprises from about 6% to about 16% metallocene-catalyzed linear low density polyethylene.
11. The polymeric composition of item 10 wherein the polymeric composition comprises about 11% metallocene-catalyzed linear low density polyethylene.
12. The polymeric composition of items 1-11 wherein the antimicrobial agent is selected from the group consisting of an organic antimicrobial agent, an inorganic antimicrobial agent, and combinations thereof.
13. The polymeric composition of item 12 wherein the antimicrobial agent is an inorganic antimicrobial agent.
14. The polymeric composition of item 13 wherein the inorganic antimicrobial agent is silver-functionalized clay.
15. The polymeric composition of item 13 wherein the inorganic antimicrobial agent includes silver ions.
16. The polymeric composition of item 12 wherein the antimicrobial agent is an organic antimicrobial agent.
17. The polymeric composition of items 1-16 wherein the antimicrobial agent promoter includes at least one blowing agent.
18. The polymeric composition of item 17 wherein the at least one blowing agent is selected from the group consisting of aliphatic or cycloaliphatic compounds, inorganic blowing agents, noble gases, and combinations thereof.
19. The polymeric composition of items 1-18 wherein the antimicrobial agent promoter includes at least one hydrophilic wetting agent.
20. The polymeric of item 19 wherein the hydrophilic wetting agent is selected from the group consisting of polyoxyalkylenes, sorbitan esters, phosphatides, alkyl amines, glycerin, water soluble polymers, surfactants, and combinations thereof.
21. The polymeric composition of items 1-20 wherein the antimicrobial agent promoter includes at least one blowing agent and at least one hydrophilic wetting agent.
22. A polymeric film comprising:
   at least one thermoplastic polymer;
   an antimicrobial agent;
      wherein the film satisfies at least one of conditions (a) and (b) in which condition (a) is that the film also comprises an antimicrobial agent promoter which includes at least one hydrophilic wetting agent, and condition (b) is that the film initially comprised an antimicrobial agent promoter that included at least one blowing agent which subsequently formed bubbles within the film and ruptured along a face of the film, to thereby form a cratered surface on the film.
23. The polymeric film of item 22 wherein the thermoplastic polymer is a sealable organic polymer.
24. The polymeric film of item 23 wherein the sealable organic polymer is ethylene vinyl acetate (EVA).
25. The polymeric film of item 24 wherein the ethylene vinyl acetate has a vinyl acetate content of 10% to 40%.
26. The polymeric film of item 24 wherein the ethylene vinyl acetate has a vinyl acetate content of 16% to 36%.
27. The polymeric film of item 24 wherein the ethylene vinyl acetate has a vinyl acetate content of 22% to 32%.
28. The polymeric film of item 24 wherein the polymeric composition comprises from about 40% to about 50% ethylene vinyl acetate.
29. The polymeric film of item 28 wherein the polymeric composition comprises about 45% ethylene vinyl acetate.
30. The polymeric film of items 22-29 further comprising linear low density polyethylene.
31. The polymeric film of item 30 wherein the polymeric composition comprises from about 6% to about 16% metallocene-catalyzed linear low density polyethylene.
32. The polymeric film of item 31 wherein the polymeric composition comprises about 11% metallocene-catalyzed linear low density polyethylene.
33. The polymeric film of items 22-32 wherein the antimicrobial agent is selected from the group consisting of an organic antimicrobial agent, an inorganic antimicrobial agent, and combinations thereof.
34. The polymeric film of item 33 wherein the antimicrobial agent is an inorganic antimicrobial agent.
35. The polymeric film of item 34 wherein the inorganic antimicrobial agent is silver-functionalized clay.
36. The polymeric film of item 34 wherein the inorganic antimicrobial agent includes silver ions.
37. The polymeric film of item 33 wherein the antimicrobial agent is an organic antimicrobial agent.
38. The polymeric film of items 22-37 wherein the film satisfies condition (b).
39. The polymeric film of item 38 wherein the at least one blowing agent is selected from the group consisting of aliphatic or cycloaliphatic compounds, inorganic blowing agents, noble gases, and combinations thereof.
40. The polymeric film of items 22-39 wherein the film satisfies condition (a).
41. The polymeric film of item 40 wherein the hydrophilic wetting agent is selected from the group consisting of polyoxyalkylenes, sorbitan esters, phosphatides, alkyl amines, glycerin, water soluble polymers, surfactants, and combinations thereof.
42. The polymeric of items 22-41 wherein the film satisfies conditions (a) and (b).
43. A method for increasing the effectiveness of an antimicrobial agent incorporated in a polymeric composition, the method comprising:
   providing a polymeric composition including at least one thermoplastic polymer, and an antimicrobial agent;
   adding to the composition an antimicrobial agent promoter selected from the group consisting of (i) at least one blowing agent, (ii) at least one hydrophilic wetting agent, and (iii) combinations of (i) and (ii) to thereby form an improved polymeric composition.
44. The method of item 43 further comprising:
   extruding the improved polymeric composition, to thereby form a polymeric film.
45. The method of items 43-44 wherein the thermoplastic polymer is a sealable organic polymer.
46. The method of item 45 wherein the sealable organic polymer is ethylene vinyl acetate (EVA).
47. The method of item 46 wherein the ethylene vinyl acetate has a vinyl acetate content of 10% to 40%.
48. The method of item 46 wherein the ethylene vinyl acetate has a vinyl acetate content of 16% to 36%.
49. The method of item 46 wherein the ethylene vinyl acetate has a vinyl acetate content of 22% to 32%.
50. The method of item 46 wherein the polymeric composition comprises from about 40% to about 50% ethylene vinyl acetate.
51. The method of item 50 wherein the polymeric composition comprises about 45% ethylene vinyl acetate.
52. The method of items 43-51 wherein the polymeric composition further includes comprising linear low density polyethylene.
53. The polymeric composition of item 52 wherein the polymeric composition comprises from about 6% to about 16% metallocene-catalyzed linear low density polyethylene.
54. The polymeric composition of item 53 wherein the polymeric composition comprises about 11% metallocene-catalyzed linear low density polyethylene.
55. The method of items 43-54 wherein the antimicrobial agent is selected from the group consisting of an organic antimicrobial agent, an inorganic antimicrobial agent, and combinations thereof.
56. The method of item 55 wherein the antimicrobial agent is an inorganic antimicrobial agent.
57. The method of item 56 wherein the inorganic antimicrobial agent is silver-functionalized clay.
58. The of item 56 wherein the inorganic antimicrobial agent includes silver ions.
59. The method of item 55 wherein the antimicrobial agent is an organic antimicrobial agent.
60. The method of items 43-59 wherein the antimicrobial agent promoter is at least one blowing agent.
61. The method of item 60 wherein the at least one blowing agent is selected from the group consisting of aliphatic or cycloaliphatic compounds, inorganic blowing agents, noble gases, and combinations thereof.
62. The method of items 43-61 wherein the antimicrobial agent promoter is at least one hydrophilic wetting agent.
63. The method of item 62 wherein the hydrophilic wetting agent is selected from the group consisting of polyoxyalkylenes, sorbitan esters, phosphatides, alkyl amines, glycerin, water soluble polymers, surfactants, and combinations thereof.
64. The method of items 43-63 wherein the antimicrobial agent promoter includes at least one blowing agent and at least one hydrophilic wetting agent.
65. A multilayer barrier film comprising:
   an odor barrier composite including layers of at least one of norbornene-based material and ethylene vinyl alcohol; and
   a polymeric film including
      at least one thermoplastic polymer;
      an antimicrobial agent;
   wherein the film satisfies at least one of conditions (a) and (b) in which condition (a) is that the film also comprises an antimicrobial agent promoter which includes at least one hydrophilic wetting agent, and condition (b) is that the film initially comprised an antimicrobial agent promoter that included at least one blowing agent which subsequently formed bubbles within the film and ruptured along a face of the film, to thereby form a cratered surface on the film.
66. The multilayer barrier film of item 65 wherein the thermoplastic polymer is a sealable organic polymer.
67. The multilayer barrier film of item 66 wherein the sealable organic polymer is ethylene vinyl acetate (EVA).
68. The multilayer barrier film of of item 67 wherein the ethylene vinyl acetate has a vinyl acetate content of 10% to 40%.
69. The multilayer barrier film of item 67 wherein the ethylene vinyl acetate has a vinyl acetate content of 16% to 36%.
70. The multilayer barrier film of item 67 wherein the ethylene vinyl acetate has a vinyl acetate content of 22% to 32%.
71. The multilayer barrier film of item 67 wherein the polymeric film comprises from about 40% to about 50% ethylene vinyl acetate.
72. The multilayer barrier of item 71 wherein the polymeric film comprises about 45% ethylene vinyl acetate.
73. The multilayer barrier film of items 65-72 wherein the polymeric film further includes linear low density polyethylene.
74. The multilayer barrier film of item 73 wherein the polymeric film comprises from about 6% to about 16% metallocene-catalyzed linear low density polyethylene.
75. The multilayer barrier film of item 74 wherein the polymeric film comprises about 11% metallocene-catalyzed linear low density polyethylene.
76. The multilayer barrier film of items 65-75 wherein the antimicrobial agent is selected from the group consisting of an organic antimicrobial agent, an inorganic antimicrobial agent, and combinations thereof.
77. The multilayer barrier film of item 76 wherein the antimicrobial agent is an inorganic antimicrobial agent.
78. The multilayer barrier film of item 77 wherein the inorganic antimicrobial agent is silver-functionalized clay.
79. The multilayer barrier film of item 77 wherein the inorganic antimicrobial agent 79. The multilayer barrier film of item 77 wherein the inorganic antimicrobial agent includes silver ions.
80. The multilayer barrier film of item 76 wherein the antimicrobial agent is an organic antimicrobial agent.
81. The multilayer barrier film of items 65-80 wherein the film satisfies condition (b).
82. The multilayer barrier of item 81 wherein the at least one blowing agent is selected from the group consisting of aliphatic or cycloaliphatic compounds, inorganic blowing agents, noble gases, and combinations thereof.
83. The multilayer barrier film of items 65-82 wherein the film satisfies condition (a).
84. The multilayer barrier film of item 83 wherein the hydrophilic wetting agent is selected from the group consisting of polyoxyalkylenes, sorbitan esters, phosphatides, alkyl amines, glycerin, water soluble polymers, surfactants, and combinations thereof.
85. The multilayer barrier film of item 65-84 wherein the film satisfies conditions (a) and (b).
86. The multilayer barrier film of items 65-85 wherein the odor barrier composite includes a cyclic olefin copolymer (COC).
87. The multilayer barrier film of item 86 wherein the cyclic olefin copolymer is a copolymer of norbornene and ethylene.
88. The multilayer barrier film of items 86-87 wherein the cyclic olefin copolymer is present in the moisture or odor barrier layer in a concentration of from about 60% to about 80%.
89. The multilayer barrier film of item 88 wherein the cyclic olefin copolymer is present in the moisture or odor barrier layer in a concentration of about 70%.
90. The multilayer barrier film of items 86-89 wherein the odor barrier composite further includes a tie component.
91. The multilayer barrier film of item 90 wherein the tie component is g-maleic anhydride ethylene vinyl acetate.

## Claims

1. A multilayered polymeric film, comprising:
a first skin layer comprising at least one gas and vapor barrier organic polymer and having an upper surface and a lower surface;
a core layer comprising at least one elastomeric organic polymer and having an upper surface and a lower surface wherein the upper surface of the core layer underlies the lower surface of the first skin layer; and
a second skin layer comprising at least one sealable organic polymer and at least one antimicrobial agent and having an upper surface and a lower surface wherein the upper surface of the second skin layer underlies the lower surface of the core layer.

2. The film of claim 1 wherein the film further comprises:
at least one tie layer comprising at least one adhesive organic polymer and having an upper surface and a lower surface wherein the upper surface of the tie layer underlies the lower surface of the first skin layer and the lower surface of the tie layer overlies the upper surface of the core layer or the upper surface of the tie layer underlies the lower surface of the core layer and the lower surface of the tie layer overlies the upper surface of the second skin layer.

3. The film of claims 1-2 wherein the gas and vapor barrier organic polymer content of the first skin layer is greater than 50% by weight.

4. The film of claims 1-3 wherein the gas and vapor barrier organic polymer of the first skin layer comprises a vinyl alcohol containing homopolymer or copolymer.

5. The film of claims 1-4 wherein the first skin layer further comprises at least one alkene-unsaturated carboxylic acid or unsaturated carboxylic acid derivative copolymer.

6. The film of claims 1-5 wherein the first skin layer further comprises at least one additive.

7. The film of claim 6 wherein the additive comprises a filler wherein the filler comprises a nanometer-sized filler.

8. The film of claims 1-7 wherein the elastomeric organic polymer of the core layer comprises a polyolefin-based elastomer.

9. The film of claims 1-8 wherein the elastomeric organic polymer of the core layer has an ASTM D 882 tensile modulus of less than 40 MPa in both the machine direction and the transverse direction and an ASTM D 882 tensile strength at break of at least 10 MPa in both the machine direction and the transverse direction.

10. The film of claims 1-9 wherein the sealable organic polymer of the second skin layer comprises an alkene-unsaturated carboxylic acid or unsaturated carboxylic acid derivative copolymer.

11. The film of claims 1-10 wherein the antimicrobial agent of the second skin layer comprises an inorganic antimicrobial agent.

12. The film of claims 1-11 wherein the antimicrobial agent of the second skin layer is delivered to the second skin layer in an additive concentrate wherein the additive concentrate comprises the antimicrobial agent and a poly(vinyl alcohol) carrier.

13. The film of claims 1-12 wherein the antimicrobial agent of the second skin layer comprises an antimicrobial agent functionalized nanometer-sized filler additive.

14. The film of claims 1-13 wherein the film has an ASTM D 882 tensile modulus of less than 138 MPa in both the machine direction and the transverse direction.

15. The film of claims 1-14 wherein the film has an ASTM D 1922 tear strength of at least 100 grams force in both the machine direction and the transverse direction.

16. The film of claims 1-15 wherein the film has an ASTM F 1927 oxygen transmission rate of less than 400 cm³/(m²-day-atm) at 37°C and 90% relative humidity.

17. The film of claims 1-16 wherein the film has an ASTM F 1249 moisture vapor transmission rate of less than 30 g/(m²-day-atm) at 37°C and 100% relative humidity.

18. An article wherein the article comprises the film of claims 1-17.

19. A method to provide a gas and vapor barrier and antimicrobial activity in an article
manufactured from a film, comprising:
providing a film wherein the film comprises a first skin layer comprising at least one gas and vapor barrier organic polymer and having an upper surface and a lower surface; a core layer comprising at least one elastomeric organic polymer and having an upper surface and a lower surface wherein the upper surface of the core layer underlies the lower surface of the first skin layer; and a second skin layer comprising at least one sealable polymer and at least one antimicrobial agent and having an upper surface and a lower surface wherein the upper surface of the second skin layer underlies the lower surface of the core layer; and
manufacturing an article wherein the article comprises the film.

20. The method of claim 19 wherein the article is used in a medical or health care application.
